# EUROPÄISCHE PATENTANMELDUNG

(11) **EP 3 786 147 A1**
(43) Veröffentlichungstag der Anmeldung: **03.03.2021**
(21) Anmeldenummer: 19194674.8
(22) Anmeldetag: 30.08.2019
(51) Int. Cl.: C07C 45/75, C07C 45/78, C07C 45/80, C07C 45/82, C07C 47/22

(54) **VERFAHREN ZUR HERSTELLUNG VON METHACROLEIN AUS FORMALDEHYD UND PROPIONALDEHYD SOWIE HERSTELLANLAGE HIERFÜR**

(71) Anmelder: Röhm GmbH, 64295 Darmstadt (DE)
(72) Erfinder: Die Erfindernennung liegt noch nicht vor
(74) Vertreter: Röhm Patent Association

(57) **Zusammenfassung**

Die vorliegende Erfindung betrifft ein Verfahren und eine Herstellanlage zum Herstellen von Methacrolein aus Formaldehyd und Propionaldehyd in Gegenwart von Wasser und in Gegenwart eines homogenen Katalysators, welcher wenigstens auf einer Säure und einer Base basiert. Damit das Verfahren mit einer guten energetischen Bilanz durchgeführt werden kann, bei welchem die anfallende Menge problematischen Abwassers möglichst gering gehalten werden kann und welches apparativ möglichst einfach umsetzbar ist, wird vorgeschlagen, Reaktionsgemisch in eine Methacroleinaufarbeitungsanlage einzuleiten und es darin in einer ersten Destillationskolonne in ein am Kopf als Gasphase vorliegendes erstes Destillationsstoffgemisch und ein im Sumpf als flüssige Phase vorliegendes zweites Destillationsstoffgemisch zu trennen, das erste Destillationsstoffgemisch zu kondensieren und in einem ersten Phasentrenner die organische Phase (erstes Trennstoffgemsich) und die wässrige Phase (zweites Trennstoffgemisch) des Kondensates voneinander zu trennen und das zweite Trennstoffgemisch in eine nicht zur Methacroleinaufarbeitungsanlage gehörende zweite Destillationskolonne einzuleiten und dort in ein am Kopf als Gasphase vorliegendes drittes Destillationsstoffgemisch sowie ein im Sumpf vorliegendes viertes Destillationsstoffgemisch zu trennen und das dritte Destillationsstoffgemisch in die Methacroleinaufarbeitungsanlage einzuleiten.

Außerdem wird eine Herstellanlage vorgeschlagen, mit welcher das erfindungsgemäße Verfahren durchgeführt werden kann.

## Beschreibung

Die vorliegende Erfindung betrifft ein Verfahren und eine Herstellanlage zum Herstellen von Methacrolein aus Formaldehyd und Propionaldehyd in Gegenwart von Wasser und in Gegenwart eines homogenen Katalysators, welcher wenigstens auf einer Säure und einer Base basiert.

Ab einer gewissen Konzentration von Katalysator im die Herstellanlage verlassenden Abwasser ist das Abwasser aus Umweltgesichtspunkten zu problematisch und kann nicht mehr zur Entsorgung in eine kommunale Kläranlage geleitet werden. Es muss entweder aufwendig behandelt werden oder einer Verbrennung zugeführt werden, um die problematischen Bestandteile in unproblematischere Stoffe umzuwandeln. Beides ist mit technischem und energetischem Aufwand verbunden.

Wenn Katalysator aus der Herstellanlage ausgeschleust wird, geht dieser der Herstellanlage verloren. Es muss dann neuer Katalysator eingesetzt werden. Dies ist mit Kosten verbunden.

Aus der DE 3213681 A1 ist ein Verfahren bekannt, bei welchem in einem Rohrreaktor unter Überdruck in flüssiger Phase aus Formaldehyd und Propionaldehyd in Gegenwart von Wasser und in Gegenwart eines homogenen Katalysators Methacrolein hergestellt wird. Das Reaktionsgemisch wird auf Normaldruck entspannt und in eine erste Destillationskolonne geleitet. Vom Kopf der ersten Destillationskolonne wird ein gasförmiger Kopfstrom, welcher Methacrolein und Wasser enthält, abgezogen. Im Sumpf sammeln sich Wasser und Katalysator. In der ersten Destillationskolonne erfolgt also das Abtrennen des homogenen Katalysators. Darüber hinaus schlägt die DE 3213681 A1 einen Reaktorbetrieb vor, welcher bei einer nur geringen Menge an Katalysator gute Reaktionsergebnisse liefert. Auf diese Weise wird schon von vornherein weniger Katalysator benötigt.

Für den Umgang mit der abgetrennten, den homogenen Katalysator enthaltenden wässrigen Phase, also dem Sumpf, schlägt die DE 3213681 A1 mehrere Varianten vor. In einer ersten Variante wird der Sumpf vollständig entsorgt. In einer zweiten Variante wird ein Teil des Sumpfes entsorgt und der übrige Teil zum Reaktor zurückgeführt. In einer dritten Variante wird der Sumpf einer zweiten Destillationskolonne zugeführt, mit welcher über Kopf Wasser destillativ abgetrennt wird. Damit ist die Konzentration des Katalysators im Sumpf der zweiten Destillationskolonne entsprechend höher als im Sumpf der ersten Destillationskolonne. Der Sumpf der zweiten Destillationskolonne wird zum Reaktor zurückgeführt. In einer vierten Variante wird nur ein Teil des Sumpfes der zweiten Destillationskolonne zum Reaktor zurückgeführt und der übrige Teil entsorgt. Die erste und zweite Variante sind, verglichen mit der dritten und vierten Variante und im Rahmen der Herstellanlage betrachtet, energetisch günstiger und apparativ deutlich einfacher. Jedoch geht mehr Katalysator verloren - womit mehr frischer Katalysator benötigt wird - als in der dritten und vierten Variante: in der ersten Variante, weil der gesamte Sumpf entsorgt wird, und in der zweiten Variante, weil nicht beliebig viel Wasser in den Reaktor zurückgeführt werden kann und daher die Menge an rückführbarem Katalysator geringer ist. Wenn der Gehalt an Katalysator im Sumpf der ersten Destillationskolonne jedoch so hoch ist, dass der Sumpf nicht als Abwasser in eine kommunale Kläranlage abgegeben werden kann, sind die zweite, dritte und vierte Variante geeignet, die Menge problematischen Abwassers, das ausgeschleust wird, gegenüber der ersten Variante zu reduzieren. In der zweiten Variante geschieht dies dadurch, dass ein Teil des Sumpfes der ersten Destillationskolonne in den Reaktor zurückgeführt wird, in der dritten Variante dadurch, dass durch die destillative Abtrennung von Wasser der gesamte Sumpf der zweiten Destillationskolonne in den Reaktor zurückgeführt werden kann und folglich kein problematisches Abwasser anfällt, und in der vierten Variante dadurch, dass durch die destillative Abtrennung von Wasser und die teilweise Rückführung des Sumpfes der zweiten Destillationskolonne in den Reaktor die Menge des problematischen Abwassers gegenüber der zweiten Variante weiter reduziert ist.

Der Kopfstrom der ersten Destillationskolonne wird in einem Kondensator kondensiert. Das Kondensat wird in einem Flüssig-flüssig-Phasentrenner in eine flüssige organische Phase, welche hauptsächlich Methacrolein enthält, und in eine flüssige wässrige Phase getrennt. Die organische Phase wird in einem Sammelbehälter als Produkt gesammelt. Die wässrige Phase wird in die erste Destillationskolonne zurückgeleitet.

Auch die WO 2016/042000 A1 beschreibt ein Verfahren, bei welchem in einem Rohrreaktor unter Überdruck in flüssiger Phase aus Formaldehyd und Propionaldehyd in Gegenwart von Wasser und in Gegenwart eines homogenen Katalysators Methacrolein hergestellt wird, das Reaktionsgemisch entspannt und in eine erste Destillationskolonne geleitet wird. Der Kopfstrom der ersten Destillationskolonne wird kondensiert. Das Kondensat wird in einen Flüssig-flüssig-Phasentrenner geleitet, in welchem eine flüssige organische Phase und eine flüssige wässrige Phase voneinander getrennt werden. Die organische Phase enthält Methacrolein in so hoher Konzentration, dass sie als Produktstrom abgeführt wird. Die wässrige Phase wird ganz oder teilweise in die erste Destillationskolonne zurückgeführt. Im Sumpf der ersten Destillationskolonne reichern sich Wasser und Katalysator an. Der Sumpf der ersten Destillationskolonne wird, von einem in die erste Kolonne zurückgeführten Anteil abgesehen, wenigstens zum Teil in den Reaktor zurückgeführt. Ein nicht in den Reaktor zurückgeführter Teil wird einer Aufarbeitung bzw. einer Entsorgung zugeleitet. Die Aufarbeitung kann in einer zweiten Destillationskolonne oder in einer Membrantrennstufe erfolgen. Im Sumpf der zweiten Destillationskolonne und im Retentat der Membrantrennstufe liegt der Katalysator in erhöhter Konzentration vor. Der Sumpf der zweiten Destillationskolonne und das Retentat der Membrantrennstufe werden in den Reaktor geleitet.

Aus der EP 2829531 A1 ist ein Verfahren bekannt, bei welchem in einem Rohrreaktor unter Überdruck in flüssiger Phase aus Formaldehyd und Propionaldehyd in Gegenwart von Wasser und in Gegenwart eines homogenen Katalysators Methacrolein hergestellt wird, das Reaktionsgemisch entspannt und in eine Destillationskolonne geleitet wird. Der Kopfstrom der Destillationskolonne wird kondensiert. Das Kondensat wird in einen Flüssig-flüssig-Phasentrenner geleitet, in welchem eine flüssige organische Phase und eine flüssige wässrige Phase voneinander getrennt werden. Die organische Phase enthält Methacrolein in so hoher Konzentration, dass sie als Produktstrom abgeführt wird. Die wässrige Phase wird in die erste Destillationskolonne zurückgeführt. Im Sumpf der ersten Destillationskolonne reichern sich Wasser und Katalysator an. Ein Teil des Sumpfstromes wird in den Reaktor zurückgeführt. Der andere Teil wird einer Aufarbeitung durch eine Membrantrennstufe zugeführt. Das Retentat, in welchem der Katalysator angereichert ist, wird wenigstens teilweise in den Reaktor zurückgeführt. Der nicht zurückgeführte Teil des Retentats wird der Entsorgung zugeführt. Das Permeat als weniger belastete wässrige Phase wird separat einer Entsorgung zugeführt.

In einer ersten Abwandlung des Verfahrens sind zwei Membrantrennstufen vorgesehen.

In einer zweiten Abwandlung des Verfahrens wird das Reaktionsgemisch stark abgekühlt und entspannt. Durch die starke Abkühlung werden Nebenreaktionen und/oder der Bildung von Di-Methacrolein deutlich reduziert. So wird Zeit für die weitere Aufarbeitung des Reaktionsgemisches gewonnen. Das abgekühlte Reaktionsgemisch wird in einen der Destillationskolonne vorgeschaltete Flüssig-flüssig-Phasentrenner geleitet, in welchem eine flüssige organische Phase und eine flüssige wässrige Phase voneinander getrennt werden. Die organische Phase wird in die Destillationskolonne geleitet und die wässrige Phase ganz oder teilweise in die Membrantrennstufe. Der Sumpf der Destillationskolonne kann in den Flüssig-flüssig-Phasentrenner, in die Membrantrennstufe oder in den Reaktor geleitet werden oder einer Entsorgung zugeführt werden.

Die WO 2018/217961 A1 offenbart ein Verfahren zum Herstellen von Methacrolein aus Formaldehyd und Propionaldehyd in Gegenwart von Wasser und in Gegenwart eines homogenen Katalysators, bei welchem die Reaktion bei Temperaturen von mehr als 100 °C, beispielsweise bei Temperaturen im Bereich von 150 - 220 °C, und Überdruck in flüssiger Phase durchgeführt wird. Das den homogenen Katalysator enthaltende Reaktionsgemisch wird stark abgekühlt, nämlich auf weniger als 15 °C, vorzugsweise auf weniger als 5 °C, und dann entspannt. Durch die starke Abkühlung werden Nebenreaktionen und/oder die Bildung von Di-Methacrolein deutlich reduziert. So wird Zeit für die weitere Aufarbeitung des Reaktionsgemisches gewonnen. Das abgekühlte Reaktionsgemisch wird in einen ersten Flüssig-flüssig-Phasentrenner geleitet, in welchem eine flüssige organische Phase, welche einen hohen Anteil an Methacrolein enthält, und eine flüssige wässrige Phase, in welcher homogener Katalysator enthalten ist, voneinander getrennt werden. Damit findet also ein erstes effektives Abtrennen von Wasser und Katalysator statt, wobei der Trennvorgang im ersten Flüssig-flüssig-Phasentrenner außer zur Flüssigkeitsförderung keine Energie benötigt. Die wässrige Phase wird in eine erste Destillationskolonne geleitet. Ein Teil des Sumpfes der ersten Destillationskolonne wird in den Reaktor zurückgeführt, ein weiterer Teil wird ausgeschleust und der verbleibende Teil wird in die erste Destillationskolonne zurückgeführt.

Von der ersten Destillationskolonne wird ein wässriger Seitenstrom abgeführt, um den Wassergehalt im Sumpf der ersten Destillationskolonne zu verringern. Dadurch steigt die Konzentration des Katalysators im Sumpf der ersten Destillationskolonne, wodurch, da nicht beliebig viel Wasser in den Reaktor zurückgeführt werden kann, die Menge des in den Reaktor zurückführbaren Katalysators erhöht wird und die Menge problematischen Abwassers, welches entsorgt werden muss, reduziert wird.

Der Kopfstrom der ersten Destillationskolonne wird kondensiert und in einen zweiten Flüssig-flüssig-Phasentrenner geleitet, in welchem eine flüssige organische Phase und eine flüssige wässrige Phase voneinander getrennt werden. Zum Auswaschen von in dem Kopfstrom der ersten Destillationskolonne enthaltenem Methanol wird dem zweiten Flüssig-flüssig-Phasentrenner zusätzliches Wasser zugeführt. Die flüssige wässrige Phase wird ausgeschleust. Die flüssige organische Phase aus dem zweiten Flüssig-flüssig-Phasentrenner und die flüssige organische Phase aus dem ersten Flüssig-flüssig-Phasentrenner werden in eine zweite Destillationskolonne geleitet. Der Kopfstrom der zweiten Destillationskolonne wird kondensiert und in den ersten Flüssig-flüssig-Phasentrenner geleitet. Der Sumpf der zweiten Destillationskolonne, welcher überwiegend Methacrolein enthält, wird, abgesehen von einem in die zweite Destillationskolonne zurückgeleiteten Teil, in eine dritte Destillationskolonne geleitet. Über den Sumpf der dritten Destillationskolonne werden unerwünschte organische Komponenten abgetrennt. Der Kopfstrom der dritten Destillationskolonne wird kondensiert und enthält Methacrolein in hoher Reinheit.

Aus der WO 2018/127963 A1 ist ein Verfahren zum Herstellen von Methacrolein aus Formaldehyd und Propionaldehyd in Gegenwart von Wasser und in Gegenwart eines homogenen Katalysators bekannt, welches sich von dem aus der WO 2018/217961 A1 bekannten Verfahren insbesondere dadurch unterscheidet, dass, der Kopfstrom der ersten Destillationskolonne nach seiner Kondensation nicht in einen zweiten Flüssig-flüssig-Phasentrenner geleitet, sondern als zweiter Produktstrom ausgeschleust wird.

Die WO 2018/217964 A1 offenbart ein Verfahren zum Herstellen von Methacrolein aus Formaldehyd und Propionaldehyd in Gegenwart von Wasser und in Gegenwart eines homogenen Katalysators, welches sich von dem aus der WO 2018/127963 A1 bekannten Verfahren insbesondere dadurch unterscheidet,
- dass der Kopfstrom der zweiten Destillationskolonne nicht anteilig in den der ersten und der zweiten Destillationskolonne vorgeschalteten Flüssig-flüssig-Phasentrenner zurückgeführt wird, sondern vollständig als erster Produktstrom ausgeschleust wird, und
- dass Sumpf der zweiten Destillationskolonne nicht in eine dritte Destillationskolonne geleitet wird, sondern ein Abfallstrom ist und ausgeschleust wird.

Aus der WO 2015/065610 A1 ist ein Verfahren zum Herstellen von Methacrolein aus Formaldehyd und Propionaldehyd in Gegenwart von Wasser und in Gegenwart eines homogenen Katalysators bekannt, welches sich von dem aus der WO 2018/127963 A1 bekannten Verfahren insbesondere dadurch unterscheidet,
- dass Sumpf der zweiten Destillationskolonne nicht in eine dritte Destillationskolonne geleitet wird, sondern bereits als Produktstrom ausgeschleust wird,
- dass ein Teil des kondensierten Kopfstromes der zweiten Destillationskolonne in die zweite Destillationskolonne zurückgeführt wird, und
- dass der Kopfstrom der ersten Destillationskolonne nicht als zweiter Produktstrom abgeführt wird, sondern teilweise in die erste Destillationskolonne und in den der ersten und der zweiten Destillationskolonne vorgeschalteten und als Dekanter ausgebildeten Flüssig-flüssig-Phasentrenner geleitet wird.

Das Verfahren kann so weitergebildet sein, dass ein Teil des kondensierten Kopfstromes der zweiten Destillationskolonne in einen separaten Dekanter geleitet wird und eine in diesem Dekanter abgetrennte flüssige organische Phase in die zweite Destillationskolonne geleitet und/oder dem Sumpfstrom der zweiten Destillationskolonne zugegeben wird. Es kann ferner dadurch weitergebildet sein, dass ein Teil des kondensierten Kopfstromes der ersten Destillationskolonne in einen weiteren separaten Dekanter geleitet wird, wobei eine in diesem Dekanter abgetrennte flüssige organische Phase in die zweite Destillationskolonne geleitet wird und eine in diesem Dekanter abgetrennte flüssige wässrige Phase in die erste Destillationskolonne geführt wird.

Der vorliegenden Erfindung liegt die Aufgabe zugrunde, ein Verfahren zur Herstellung von Methacrolein aus Formaldehyd und Propionaldehyd in Gegenwart von Wasser und in Gegenwart eines Katalysators, welcher wenigstens auf einer Säure und einer Base basiert, zu schaffen, welches mit einer guten energetischen Bilanz durchgeführt werden kann, bei welchem die anfallende Menge problematischen Abwassers möglichst gering gehalten werden kann und welches apparativ möglichst einfach umsetzbar ist. Der Erfindung liegt ferner die Aufgabe zugrunde, eine entsprechende Herstellanlage zu schaffen.

Die Verfahrensaufgabe wird erfindungsgemäß gelöst mit einem Verfahren mit den Merkmalen des Anspruches 1. Durch das Einleiten zweiten Trennstoffgemisches in die zweite Destillationskolonne wird der ersten Destillationskolonne - verglichen mit dem Fall, dass das zweite Trennstoffgemisch in die erste Destillationskolonne eingeleitete werden würde, - weniger Wasser zugeführt. Damit fällt eine entsprechend geringere Menge zweiten Destillationsstoffgemisches an und das zweite Destillationsstoffgemisch hat eine entsprechend höhere Konzentration an Katalysator. Wegen der höheren Konzentration an Katalysator kann, da die Menge des in den Reaktor zurückführbaren Wassers begrenzt ist, mehr Katalysator in den Reaktor zurückgeführt werden, wodurch frischer Katalysator eingespart wird.

Da eine geringere Menge zweiten Destillationsstoffgemisches anfällt, ist die Menge potenziell zu entsorgenden problematischen Abwassers reduziert.

Durch das Einleiten eines Teiles des zweiten Destillationsstoffgemisches in den Reaktor wird der in diesem Teil des zweiten Destillationsstoffgemisches enthaltene Katalysator wieder genutzt.

Mittels des ersten Phasentrenners wird auf einfache und effektive Weise zweites Trennstoffgemisch, und damit viel Wasser, von erstem Trennstoffgemisch, welches einen hohen Anteil an Methacrolein enthalten kann, abgetrennt.

Mit der zweiten Destillationskolonne kann noch ein guter Teil von in dem zweiten Trennstoffgemisch enthaltenem Methacrolein abgetrennt werden.

Durch das Einleiten von drittem Destillationsstoffgemisch in die Methacroleinaufarbeitungsanlage wird das dritte Destillationsstoffgemisch, welches eine methacroleinhaltige Phase ist, einer weiteren Aufarbeitung zugeführt, bei welcher die Menge an Begleitkomponenten verringert wird.

Die Menge an im vierten Destillationsstoffgemisch enthaltenem Katalysator kann klein gehalten werden, was im Hinblick auf die Entsorgung von viertem Destillationsstoffgemisch vorteilhaft ist.

Vorteilhafterweise kann in Schritt S6 wenigstens ein Teil des dritten Destillationsstoffgemisches in den ersten Kondensator eingeleitet werden. Hierdurch werden kein eigener Kondensator und Phasentrenner für das dritte Destillationsstoffgemisch benötigt und der apparative Aufwand ist insofern reduziert. Im ersten Kondensator geht das dritte Destillationsstoffgemisch in dessen Kondensat auf, welches in den ersten Phasentrenner geleitet wird.

Besonders bevorzugt kann in Schritt S6 wenigstens ein Teil des dritten Destillationsstoffgemisches in einen zur Methacroleinaufarbeitungsanlage gehörenden zweiten Kondensator eingeleitet und in diesem kondensiert werden und Kondensat aus dem zweiten Kondensator abgeführt und in den ersten Phasentrenner eingeleitet werden. Mit dem zweiten Kondensator kann das dritte Destillationsstoffgemisch unabhängig vom ersten Kondensator kondensiert werden. Zudem kann der zweite Kondensator für ein wenigstens teilweises Rezyklieren von drittem Destillationsstoffgemisch in die zweite Destillationskolonne genutzt werden. Durch das Einleiten des Kondensates des zweiten Kondensators in den ersten Phasentrenner wird kein eigener Phasentrenner für das Kondensat des zweiten Kondensators benötigt und der apparative Aufwand ist insofern reduziert.

Besonders vorteilhaft kann in Schritt S6 wenigstens ein Teil des dritten Destillationsstoffgemisches in einen zur Methacroleinaufarbeitungsanlage gehörenden zweiten Kondensator eingeleitet und in diesem kondensiert werden, Kondensat aus dem zweiten Kondensator abgeführt und in einen zur Methacroleinaufarbeitungsanlage gehörenden zweiten Phasentrenner eingeleitet und in dem zweiten Phasentrenner in ein drittes Trennstoffgemisch und ein viertes Trennstoffgemisch getrennt werden, wobei das dritte Trennstoffgemisch als organische Phase vorliegt, welche Methacrolein enthält, und das vierte Trennstoffgemisch als wässrige Phase. Durch die mittels des zweiten Phasentrenner durchgeführte Stofftrennung kann das dritte Trennstoffgemisch als zum ersten Trennstoffgemisch separater Produktstrom von gut aufgearbeitetem Methacrolein genutzt oder mit dem ersten Trennstoffgemisch zu einem gemeinsamen Produktstrom von gut aufgearbeitetem Methacrolein vereinigt werden. D. h. das dritte Trennstoffgemisch muss nicht notwendigerweise in den ersten Phasentrenner eingeleitet werden.

Außerdem besteht mit dem zweiten Kondensator und dem zweiten Phasentrenner eine besondere Möglichkeit, eine Fraktion des dritten Destillationsstoffgemisches in die zweite Destillationskolonne zurückzuführen, nämlich das vierte Trennstoffgemisch, und hierdurch eine besonders gute Stofftrennung in der zweiten Destillationskolonne durchzuführen, insbesondere eine besonders gute Trennung von Methacrolein einerseits und Wasser andererseits.

Besonders günstig kann in Schritt S6 wenigstens ein Teil des dritten Destillationsstoffgemisches in die erste Destillationskolonne eingeleitet werden. Auf diese Weise wird das dritte Destillationsstoffgemisch insbesondere der mittels der ersten Destillationskolonne und dem ersten Phasentrenner durchgeführten Aufarbeitung zugeführt.

Bevorzugterweise kann
- das Reaktionsgemisch entspannt werden, wodurch ein erstes Entspannungsstoffgemisch entsteht, welches die durch die Entspannung in die Gasphase übergehende Fraktion des Reaktionsgemisches ist, und ein zweites Entspannungsstoffgemisch, welches die in flüssiger Phase verbleibende Fraktion des Reaktionsstoffgemisches ist,
- erstes und zweites Entspannungsstoffgemisch in einem zur Methacroleinaufarbeitungsanlage gehörenden Entspannungsbehälter getrennt werden,
- das erste Entspannungsstoffgemisch in den ersten Kondensator eingeleitet werden und
- das zweite Entspannungsstoffgemisch in die erste Destillationskolonne eingeleitet werden.

Das den Reaktor verlassende Reaktionsgemisch ist flüssig und steht unter Druck. Wenn es entspannt wird, geht durch das Entspannen eine Fraktion des Reaktionsgemisches in die Gasphase über, womit das erste Entspannungsstoffgemisch, also die durch die Entspannung in die Gasphase übergehende Fraktion des Reaktionsgemisches, und das zweite Entspannungsstoffgemisch, also die in der flüssigen Phase verbleibende Fraktion des Reaktionsgemisches, entstehen. Der Methacroleinanteil im ersten Entspannungsstoffgemisch ist höher als im zweiten Entspannungsstoffgemisch. Folglich ist die Entspannung des Druckes bereits ein Schritt zum Gewinnen eines Stoffgemisches mit höherem Methacroleingehalt. Das erste Entspannungsstoffgemisch und das zweite Entspannungsstoffgemisch werden in dem Entspannungsbehälter voneinander getrennt.

Mit der Entspannung des Druckes geht bereits eine Abkühlung einher. Für diese Abkühlung muss also kein zusätzlicher energetischer oder apparativer Aufwand mehr betrieben werden. Zudem bleibt die dem Druckabbau entsprechende Energiemenge trotz der mit dem Druckabbau verbundenen Abkühlung im System.

Nur das zweite Entspannungsstoffgemisch muss in die erste Destillationskolonne geleitet und für den Destillationsvorgang erwärmt werden. Das erste Entspannungsstoffgemisch wird gleich in den ersten Kondensator geleitet und dort zusammen mit dem vom Kopf der ersten Destillationskolonne kommenden ersten Destillationsstoffgemisch kondensiert.

Günstigerweise kann in Schritt S6 wenigstens ein Teil des dritten Destillationsstoffgemisches in den Entspannungsbehälter eingeleitet werden. Auf diese Weise wird das dritte Destillationsstoffgemisch über den Weg über den Entspannungsbehälter insbesondere der mittels des ersten Phasentrenners durchgeführten Aufarbeitung zugeführt.

Besonders vorteilhaft kann wenigstens ein Teil des in Schritt S3.iv abgeführten zweiten Destillationsstoffgemisches in eine Membrananlage eingeleitet werden, in der Membrananlage wenigstens ein Teil von in dem eingeleiteten zweiten Destillationsstoffgemisch enthaltenem Katalysator zurückgehalten werden und ein Retentatstoffgemisch, welches den zurückgehaltenen Katalysator enthält, sowie ein Permeatstoffgemisch aus der Membrananlage abgeführt werden. Da das Retentatstoffgemisch eine höhere Konzentration an Katalysator hat als das zweite Destillationsstoffgemisch und die Menge des in den Reaktor zurückführbaren Wassers begrenzt ist, kann mehr Katalysator in den Reaktor zurückgeführt werden, wenn Retentatstoffgemisch für die Rückführung von Katalysator in den Reaktor verwendet wird.

Da die Menge des Retentatstoffgemisches geringer ist als die Menge des der Membrananlage zugeführten zweiten Destillationsstoffgemisches, ist die Menge potenziell zu entsorgenden problematischen Abwassers reduziert, wenn das Permeatstoffgemisch nicht als problematisches Abwasser einzustufen ist. Falls das Permeatstoffgemisch als problematisches Abwasser einzustufen ist, ist dessen Aufarbeitung insbesondere wegen des reduzierten Gehaltes an Katalysator möglicherweise zumindest einfacher.

Die Herstellaufgabe wird erfindungsgemäß gelöst mit einer Herstellanlage mit den Merkmalen des Anspruches 10. Durch die zweite Phasentrennerabführleitungsanordnung kann zweites Trennstoffgemisch in die zweite Destillationskolonne eingeleitet werden. Wenn dies erfolgt, wird der ersten Destillationskolonne weniger Wasser zugeführt als wenn diese Menge zweiten Trennstoffgemisches in die erste Destillationskolonne geleitet werden würde. Damit fällt in der ersten Destillationskolonne eine entsprechend geringere Menge zweiten Destillationsstoffgemisches an und das zweite Destillationsstoffgemisches hat eine entsprechend höhere Konzentration an Katalysator. Wegen der höheren Konzentration an Katalysator kann, da die Menge des in den Reaktor zurückführbaren Wassers begrenzt ist, mehr Katalysator in den Reaktor zurückgeführt werden. Da eine geringere Menge zweiten Destillationsstoffgemisches anfällt, ist die Menge potenziell zu entsorgenden problematischen Abwassers reduziert.

Mittels des ersten Phasentrenners wird auf einfache und effektive Weise zweites Trennstoffgemisch, und damit viel Wasser, von erstem Trennstoffgemisch, welches einen hohen Anteil an Methacrolein enthalten kann, abgetrennt.

Mit der zweiten Destillationskolonne kann noch ein guter Teil von in dem zweiten Trennstoffgemisch enthaltenem Methacrolein abgetrennt werden.

Durch die dritte Destillationskolonnenabführleitungsanordnung kann drittes Destillationsstoffgemisch in die Methacroleinaufarbeitungsanlage geleitet werden. Damit kann das dritte Destillationsstoffgemisch, welches eine methacroleinhaltige Phase ist, einer weiteren Aufarbeitung, bei welcher die Menge an Begleitkomponenten verringert wird, zugeführt werden.

Da der zweiten Destillationskolonne zweites Trennstoffgemisch zugeführt wird, dessen Menge an Katalysator klein ist, ist die Menge an im vierten Destillationsstoffgemisch enthaltenem Katalysator klein. Dies ist im Hinblick auf die Entsorgung von viertem Destillationsstoffgemisch vorteilhaft.

Vorteilhafterweise kann die dritte Destillationskolonnenabführleitungsanordnung den Kopf der zweiten Destillationskolonne mit dem ersten Kondensator fluidisch verbinden und drittes Destillationsstoffgemisch durch die dritte Destillationskolonnenabführleitungsanordnung vom Kopf der zweiten Destillationskolonne in den ersten Kondensator geleitet werden. Da drittes Destillationsstoffgemisches in den ersten Kondensator eingeleitet werden kann, werden kein eigener Kondensator und Phasentrenner für das dritte Destillationsstoffgemisch benötigt und der apparative Aufwand ist insofern reduziert. Im ersten Kondensator geht das dritte Destillationsstoffgemisch in dessen Kondensat auf, welches in den ersten Phasentrenner geleitet werden kann.

Vorzugsweise kann
- die Herstellanlage einen zur Methacroleinaufarbeitungsanlage gehörenden zweiten Kondensator aufweisen,
- die dritte Destillationskolonnenabführleitungsanordnung den Kopf der zweiten Destillationskolonne mit dem zweiten Kondensator fluidisch verbinden, wobei drittes Destillationsstoffgemisch durch die dritte Destillationskolonnenabführleitungsanordnung vom Kopf der zweiten Destillationskolonne in den zweiten Kondensator geleitet und in dem zweiten Kondensator kondensiert werden kann, und
- der zweite Kondensator fluidisch mit dem ersten Phasentrenner verbunden sein, wodurch Kondensat des zweiten Kondensators vom zweiten Kondensator weiter in den ersten Phasentrenner geleitet werden kann.

Mit dem zweiten Kondensator kann das dritte Destillationsstoffgemisch unabhängig vom ersten Kondensator kondensiert werden. Zudem kann der zweite Kondensator für ein wenigstens teilweises Rezyklieren dritten Destillationsstoffgemisches in die zweite Destillationskolonne genutzt werden.

Außerdem ist mit diesem Aufbau kein eigener Phasentrenner für das kondensierte Destillationsstoffgemisch erforderlich. Insofern ist der apparative Aufwand reduziert.

Besonders bevorzugt kann
- die Herstellanlage einen zur Methacroleinaufarbeitungsanlage gehörenden zweiten Kondensator aufweisen,
- die dritte Destillationskolonnenabführleitungsanordnung den Kopf der zweiten Destillationskolonne mit dem zweiten Kondensator fluidisch verbinden, wobei drittes Destillationsstoffgemisch durch die dritte Destillationskolonnenabführleitungsanordnung vom Kopf der zweiten Destillationskolonne in den zweiten Kondensator geleitet und in dem zweiten Kondensator kondensiert werden kann, und
- der zweite Kondensator fluidisch mit einem zur Methacroleinaufarbeitungsanlage gehörenden zweiten Phasentrenner verbunden sein, wodurch Kondensat des zweiten Kondensators vom zweiten Kondensator weiter in den zweiten Phasentrenner geleitet werden kann, mit welchem ein im vom zweiten Kondensator zugeführten Kondensat enthaltenes drittes Trennstoffgemisch organischer Phase von einem im vom zweiten Kondensator zugeführten Kondensat enthaltenen vierten Trennstoffgemisch wässriger Phase getrennt werden kann, wobei der zweite Phasentrenner einen ersten Ausgang zum Abführen von drittem Trennstoffgemisch und einen zweiten Ausgang zum Abführen von viertem Trennstoffgemisch aufweist.

Mit diesem Aufbau kann wenigstens ein Teil des dritten Destillationsstoffgemisches in den zweiten Kondensator eingeleitet und in diesem kondensiert werden, Kondensat aus dem zweiten Kondensator abgeführt und in den zweiten Phasentrenner eingeleitet werden, in welchem das dritte Trennstoffgemisch und das vierte Trennstoffgemisch getrennt werden können. Durch die vom zweiten Phasentrenner durchgeführte Stofftrennung kann das dritte Trennstoffgemisch als zum ersten Trennstoffgemisch separater Produktstrom von gut aufgearbeitetem Methacrolein genutzt oder mit dem ersten Trennstoffgemisch zu einem gemeinsamen Produktstrom von gut aufgearbeitetem Methacrolein vereinigt werden. D. h. das dritte Trennstoffgemisch muss nicht notwendigerweise in den ersten Phasentrenner eingeleitet werden. Außerdem besteht mit dem zweiten Kondensator und dem zweiten Phasentrenner eine besondere Möglichkeit, eine Fraktion des dritten Destillationsstoffgemisches in die zweite Destillationskolonne zurückzuführen, nämlich das vierte Trennstoffgemisch, und hierdurch eine besonders gute Stofftrennung in der zweiten Destillationskolonne durchzuführen, insbesondere eine gute Trennung von Methacrolein einerseits und Wasser andererseits.

Besonders günstig kann die dritte Destillationskolonnenabführleitungsanordnung den Kopf der zweiten Destillationskolonne mit der ersten Destillationskolonne fluidisch verbinden und drittes Destillationsstoffgemisch durch die dritte Destillationskolonnenabführleitungsanordnung vom Kopf der zweiten Destillationskolonne in die erste Destillationskolonne geleitet werden. So kann drittes Destillationsstoffgemisch insbesondere der mit dem ersten Phasentrenner durchgeführten Aufarbeitung zugeführt werden.

Bevorzugterweise kann die Methacroleinaufarbeitungsanlage einen Entspannungsbehälter aufweisen, mit welchem ein erstes Entspannungsstoffgemisch, welches die durch eine Entspannung des Reaktionsgemisches in die Gasphase übergehende Fraktion des Reaktionsgemisches ist, und ein zweites Entspannungsstoffgemisch, welches die nach der Entspannung des Reaktionsgemisches in flüssiger Phase verbleibende Fraktion des Reaktionsgemisches ist, voneinander getrennt werden können, wobei der Entspannungsbehälter einen ersten Ausgang zum Abführen von erstem Entspannungsstoffgemisch hat, welcher mit dem ersten Kondensator fluidisch verbunden ist, wodurch erstes Entspannungsstoffgemisch in den ersten Kondensator geleitet werden kann, und einen zweiten Ausgang zum Abführen von zweitem Entspannungsstoffgemisch, welcher mit der ersten Destillationskolonne fluidisch verbunden ist, wodurch zweites Entspannungsstoffgemisch in die erste Destillationskolonne geleitet werden kann. Das den Reaktor verlassende Reaktionsgemisch ist flüssig und steht unter Druck. Wenn es entspannt wird, geht durch das Entspannen eine Fraktion des Reaktionsgemisches in die Gasphase über, womit das erste Entspannungsstoffgemisch, also die durch die Entspannung in die Gasphase übergehende Fraktion des Reaktionsgemisches, und das zweite Entspannungsstoffgemisch, also die in der flüssigen Phase verbleibende Fraktion des Reaktionsgemisches, entstehen. Der Methacroleinanteil im ersten Entspannungsstoffgemisch ist höher als im zweiten Entspannungsstoffgemisch. Folglich ist die Entspannung des Druckes bereits ein Schritt zum Gewinnen eines Stoffgemisches mit höherem Methacroleingehalt. Mit dem Entspannungsbehälter können das erste und das zweite Entspannungsstoffgemisch voneinander getrennt werden und beide können getrennt aus dem Entspannungsbehälter abgeführt werden. Dadurch, dass der erste Ausgang fluidisch mit dem ersten Kondensator verbunden ist, kann erstes Entspannungsstoffgemisch in den ersten Kondensator geleitet werden. Dort kann es zusammen mit dem vom Kopf der ersten Destillationskolonne kommenden ersten Destillationsstoffgemisch kondensiert werden. Dadurch, dass der zweite Ausgang fluidisch mit der ersten Destillationskolonne verbunden ist, kann zweites Entspannungsstoffgemisch in die erste Destillationskolonne geleitet werden - nur das zweite Entspannungsstoffgemisch muss in die erste Destillationskolonne geleitet werden und dementsprechend für den Destillationsvorgang erwärmt werden.

Günstigerweise kann die dritte Destillationskolonnenabführleitungsanordnung den Kopf der zweiten Destillationskolonne mit dem Entspannungsbehälter fluidisch verbinden und drittes Destillationsstoffgemisch kann durch die dritte Destillationskolonnenabführleitungsanordnung vom Kopf der zweiten Destillationskolonne in den Entspannungsbehälter geleitet werden. Hierdurch kann das dritte Destillationsstoffgemisch über den Weg über den Entspannungsbehälter insbesondere der mittels des ersten Phasentrenners durchgeführten Aufarbeitung zugeführt werden.

Vorteilhafterweise kann der Sumpf der ersten Destillationskolonne mit dem Reaktor fluidisch verbunden sein, wodurch zweites Destillationsstoffgemisch in den Reaktor geleitet werden kann. Durch diesen Aufbau kann in zweitem Destillationsstoffgemisch enthaltener Katalysator wieder genutzt werden.

Bevorzugt kann die Herstellanlage eine mit dem Sumpf der ersten Destillationskolonne fluidisch verbundene Membrananlage aufweisen, mit welcher wenigstens ein Teil von in der Membrananlage zugeleitetem zweiten Destillationsstoffgemisch enthaltenem Katalysator zurückgehalten werden kann, und die Membrananlage kann einen ersten Ausgang zum Abführen von den zurückgehaltenen Katalysator enthaltendem Retentatstoffgemisch aufweisen sowie einen zweiten Ausgang zum Abführen von Permeatstoffgemisch. Mit Hilfe der Membrananlage wird es möglich, mehr Katalysator in den Reaktor zurückzuführen. Denn die Menge des in den Reaktor zurückführbaren Wassers ist begrenzt. Das Retentatstoffgemisch hat eine höhere Konzentration an Katalysator als das zweite Destillationsstoffgemisch und kann für die Rückführung von Katalysator in den Reaktor verwendet werden.

Zudem kann durch den Einsatz der Membrananlage die Menge des potenziell zu entsorgenden problematischen Abwassers reduziert werden, wenn das Permeatstoffgemisch nicht als problematisches Abwasser einzustufen ist. Denn die Menge den Retentatstoffgemisches ist geringer als die Menge des der Membrananlage zugeführten zweiten Destillationsstoffgemisches. Falls das Permeatstoffgemisch als problematisches Abwasser einzustufen ist, ist dessen Aufarbeitung insbesondere wegen des reduzierten Gehaltes an Katalysator möglicherweise zumindest einfacher.

Besonders bevorzugt kann die Herstellanlage in allen Varianten, die mit den beschriebenen Ausgestaltungsmöglichkeiten möglich sind, zum Herstellen von Methacrolein, insbesondere zum Herstellen von Methacrolein aus Formaldehyd und Propionaldehyd in Gegenwart von Wasser und in Gegenwart eines homogenen Katalysators, welcher wenigstens auf einer Säure und einer Base basiert, verwendet werden.

In den Figuren sind einige mögliche Ausführungsformen der vorliegenden Erfindung gezeigt. Es zeigen:
Figur 1 eine ersten Ausführungsform der Erfindung anhand eines schematischen Fließschemas,
Figur 2 eine zweite Ausführungsform der Erfindung anhand eines schematischen Fließschemas,
Figur 3 eine dritte Ausführungsform der Erfindung anhand eines schematischen Fließschemas,
Figur 4 eine vierte Ausführungsform der Erfindung anhand eines schematischen Fließschemas,
Figur 5 eine fünfte Ausführungsform der Erfindung anhand eines schematischen Fließschemas,
Figur 6 eine sechste Ausführungsform der Erfindung anhand eines schematischen Fließschemas,
Figur 7 eine siebente Ausführungsform der Erfindung anhand eines schematischen Fließschemas,
Figur 8 eine achte Ausführungsform der Erfindung anhand eines schematischen Fließschemas und
Figur 9 eine neunte Ausführungsform der Erfindung anhand eines schematischen Fließschemas.

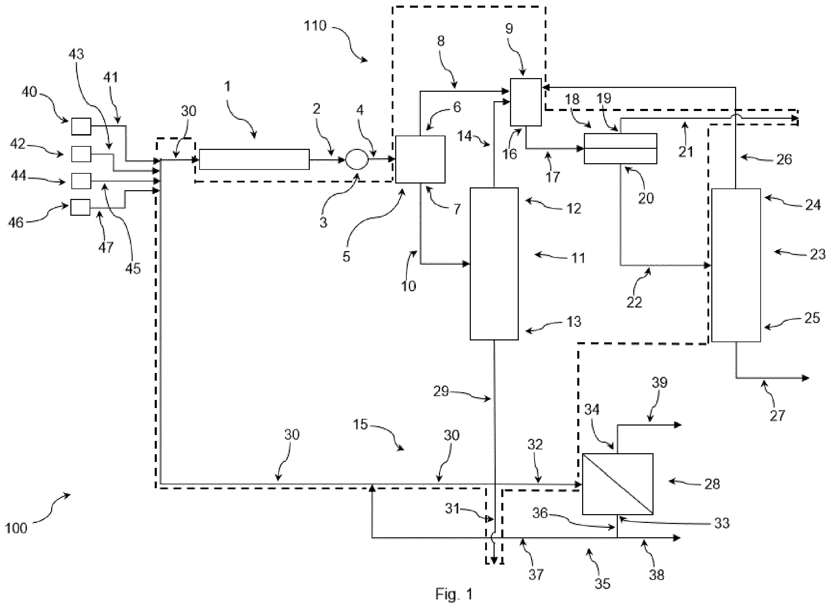

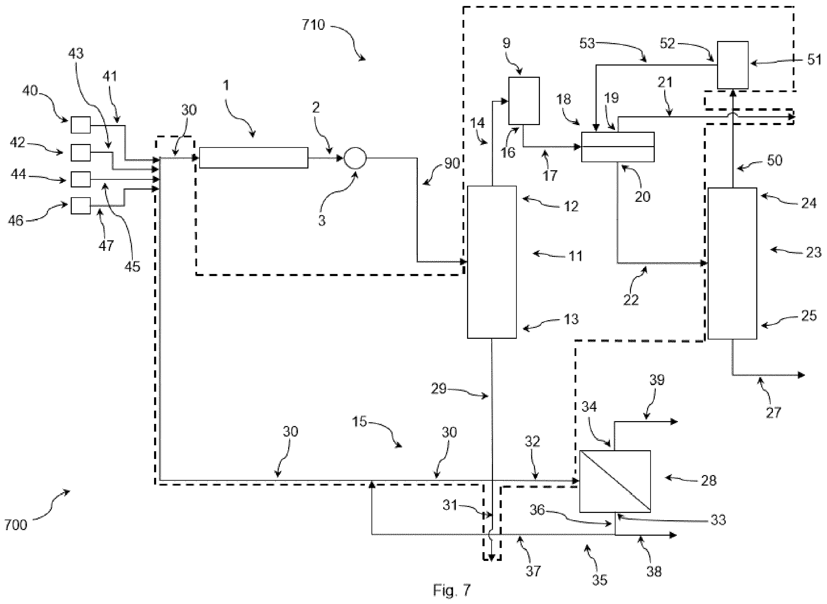

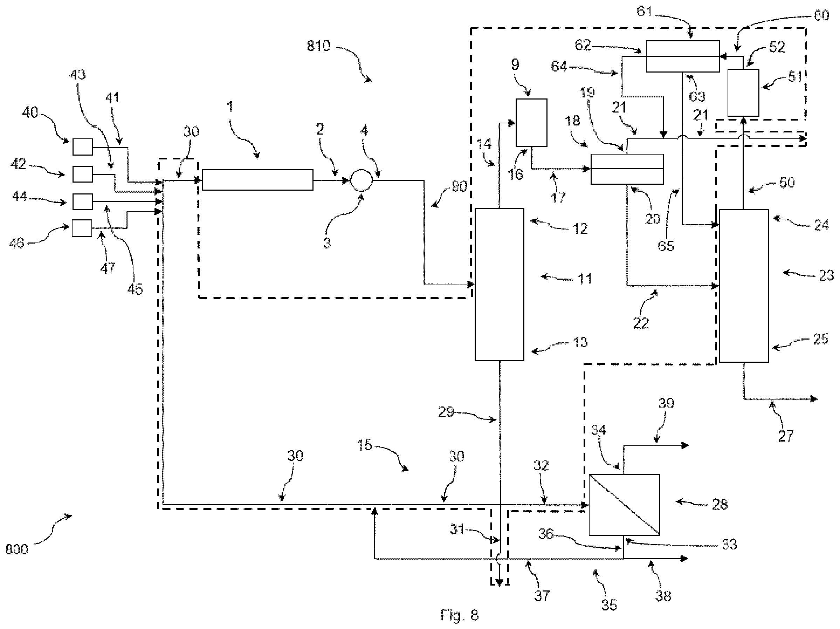

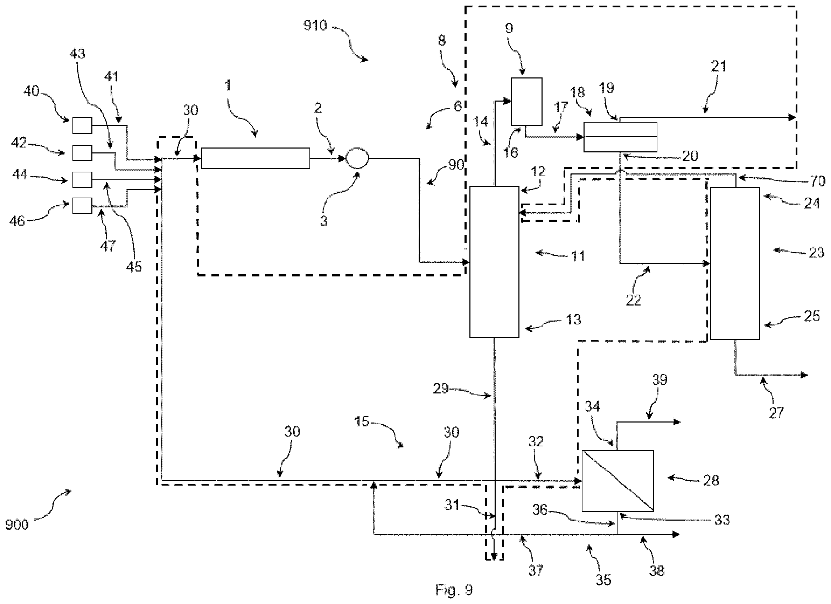

In den schematischen Fließschemata in den Figuren 1 bis 9 zeigen die Pfeile die Fließrichtung des jeweiligen Mediums an, welches es beim Durchführen des betreffenden Verfahrens hat. Ließe man die Pfeile weg, wären die Figuren 1 bis 9 schematische Darstellungen der Herstellanlagen der ersten bis neunten Ausführungsform der Erfindung.

In den Figuren 1 bis 9 ist durch gestrichelte Linien angedeutet, welcher Teil der jeweiligen Herstellanlage zur Methacroleinaufbereitungsanlage dieser Herstellanlage gehört.

Es wird Bezug auf Figur 1 genommen. Die darin veranschaulichte Herstellanlage 100 der ersten Ausführungsform der Erfindung weist einen Reaktor 1 auf, in welchem eine Reaktion in der Flüssigphase unter Überdruck durchgeführt werden kann, bei welcher aus Formaldehyd und Propionaldehyd in Gegenwart von Wasser und in Gegenwart eines homogenen Katalysators, welcher wenigstens auf einer Säure und einer Base basiert, Methacrolein gebildet wird. Eine Reaktorabführleitungsanordnung 2 verbindet den Reaktor 1 mit einem Entspannungsventil 3 fluidisch, wodurch Reaktionsstoffgemisch vom Reaktor 1 durch die Reaktorabführleitungsanordnung 2 zum Entspannungsventil 3 geleitet werden kann. Mit dem Entspannungsventil 3 kann vom Reaktor 1 kommendes und unter Überdruck stehendes Reaktionsgemisch auf einen niedrigeren Druck entspannt werden, wobei mit dem Entspannungsventil 3 gleichzeitig der Druck im Reaktor 1 reguliert werden kann. Man könnte das Entspannungsventil 3 auch als Druckhalte- und Entspannungsventil bezeichnen.

Das Entspannungsventil 3 ist fluidisch mit einer Methacroleinaufbereitungsanlage 110 verbunden, mit welcher der Anteil, welchen Begleitkomponenten, beispielsweise Wasser, Katalysator, nicht umgesetztes Formaldehyd und nicht umgesetztes Propionaldehyd, am Reaktionsgemisch oder an einer Fraktion des Reaktionsgemisches ausmachen, verringert werden kann. Zu der Methacroleinaufbereitungsanlage 110 gehört ein Entspannungsbehälter 5. Mit diesem ist das Entspannungsventil 3 über eine Entspannungsventilabführleitungsanordnung 4 fluidisch verbunden. Mit dem Entspannungsbehälter 5 kann ein erstes Entspannungsstoffgemisch, welches die durch eine Entspannung von Reaktionsgemisch in die Gasphase übergehende Fraktion des Reaktionsgemisches ist, und ein zweites Entspannungsstoffgemisch, welches die nach der Entspannung des Reaktionsgemisches in flüssiger Phase verbleibende Fraktion des Reaktionsgemisches ist, voneinander getrennt werden. Der Entspannungsbehälter 5 kann auch als Entspannungstrommel gezeichnet werden, wobei er keine Trommelform haben muss, oder als Flashbox.

Der Entspannungsbehälter 5 hat einen ersten Ausgang 6 zum Abführen von erstem Entspannungsstoffgemisch und einen zweiten Ausgang 7 zum Abführen von zweitem Entspannungsstoffgemisch.

Die Methacroleinaufbereitungsanlage 110 weist ferner einen ersten Kondensator 9 auf. Der erste Ausgang 6 des Entspannungsbehälters 5 ist über eine erste Entspannungsbehälterabführleitungsanordnung 8 fluidisch dem ersten Kondensator 9 verbunden, durch welche erstes Entspannungsstoffgemisch von dem Entspannungsbehälter 5 in den ersten Kondensator 9 geleitet werden kann. Mit dem ersten Kondensator 9 kann erstes Entspannungsstoffgemisch kondensiert werden.

Die Methacroleinaufbereitungsanlage 110 weist außerdem eine erste Destillationskolonne 11 auf, welche einen Kopf 12 und einen Sumpf 13 hat. Der zweiter Ausgang 7 des Entspannungsbehälters 5 ist über eine zweite Entspannungsbehälterabführleitungsanordnung 10 fluidisch mit der ersten Destillationskolonne 11 verbunden, wobei zweites Entspannungsstoffgemisch durch die zweite Entspannungsbehälterabführleitungsanordnung 10 von dem Entspannungsbehälter 5 in die erste Destillationskolonne 11 geleitet werden kann. Mit der ersten Destillationskolonne 11 kann zweites Entspannungsstoffgemisch in ein als Gasphase vorliegendes erstes Destillationsstoffgemisch, welches Methacrolein enthält, und ein als flüssige Phase vorliegendes zweites Destillationsstoffgemisch, welches Wasser und Katalysator enthält, getrennt werden.

Über eine erste Destillationskolonnenabführleitungsanordnung 14 ist der Kopf 12 der ersten Destillationskolonne 11 fluidisch mit dem ersten Kondensator 9 verbunden. Durch die erste Destillationskolonnenabführleitungsanordnung 14 kann erstes Destillationsstoffgemisch vom Kopf 12 der ersten Destillationskolonne 11 in den ersten Kondensator 9 geleitet werden. In dem ersten Kondensator 9 kann das zugeführte erste Destillationsstoffgemisches kondensiert werden.

Die erste Entspannungsbehälterabführleitungsanordnung 8 und die erste Destillationskolonnenabführleitungsanordnung 14 sind mit je einem eigenen Eingang des ersten Kondensators 9 verbunden sein.

Der Sumpf 13 der ersten Destillationskolonne 11 ist durch eine zweite Destillationskolonnenabführleitungsanordnung 15 mit dem Reaktor 1 fluidisch verbunden. Durch die zweite Destillationskolonnenabführleitungsanordnung 15 kann zweites Destillationsstoffgemisch aus der ersten Destillationskolonne 11 in den Reaktor 1 geleitet werden.

Die Methacroleinaufbereitungsanlage 110 weist ferner einen ersten Phasentrenner 18 auf. Der erste Phasentrenner 18 ist über eine Kondensatorabführleitungsanordnung 17 mit einem Kondensatausgang 16 des ersten Kondensators 9 fluidisch verbunden. Durch die Kondensatorabführleitungsanordnung 17 kann Kondensat des ersten Kondensators 9 vom ersten Kondensator 9 in den ersten Phasentrenner 18 geleitet werden. Mit dem ersten Phasentrenner 18 kann ein im Kondensat des ersten Kondensators 9 enthaltenes erstes Trennstoffgemisch organischer Phase von einem im Kondensat des ersten Kondensators 9 enthaltenen zweiten Trennstoffgemisch wässriger Phase getrennt werden. Der erste Phasentrenner 18 weist einen ersten Ausgang 19 zum Abführen von erstem Trennstoffgemisch und einen zweiten Ausgang 20 zum Abführen von zweitem Trennstoffgemisch auf. An den ersten Ausgang 19 des ersten Phasentrenners 18 ist eine erste Phasentrennerabführleitungsanordnung 21 fluidisch angeschlossen, durch welche erstes Trennstoffgemisch aus dem ersten Phasentrenner 18 abgeführt werden kann. An den zweiten Ausgang 20 ist eine zweite Phasentrennerabführleitungsanordnung 22 fluidisch angeschlossen, durch welche zweites Trennstoffgemisch aus dem ersten Phasentrenner 18 abgeführt werden kann.

Die Herstellanlage 100 weist ferner eine zweite Destillationskolonne 23 auf, welche einen Kopf 24 und einen Sumpf 25 hat. Die zweite Destillationskolonne 23 gehört nicht zu der Methacroleinaufbereitungsanlage 110. Über die zweite Phasentrennerabführleitungsanordnung 22 ist der zweite Ausgang 20 des ersten Phasentrenners 18 mit der zweiten Destillationskolonne 23 fluidisch verbunden. Über die zweite Phasentrennerabführleitungsanordnung 22 kann zweites Trennstoffgemisch vom zweiten Ausgang des ersten Phasentrenners 18 in die zweite Destillationskolonne 23 geleitet werden. Mit der zweiten Destillationskolonne 23 kann zweites Trennstoffgemisch wenigstens in ein als Gasphase vorliegendes und Methacrolein enthaltendes drittes Destillationsstoffgemisches und ein als flüssige Phase vorliegendes und Wasser enthaltendes viertes Destillationsstoffgemisches getrennt werden.

An den Kopf 24 der zweiten Destillationskolonne 23 ist eine dritte Destillationskolonnenabführleitungsanordnung 26 fluidisch angeschlossen. Über die dritte Destillationskolonnenabführleitungsanordnung 26 ist der Kopf 24 der zweiten Destillationskolonne 23 mit der Methacroleinaufbereitungsanlage 110 fluidisch verbunden, wobei drittes Destillationsstoffgemisch durch die dritte Destillationskolonnenabführleitungsanordnung 26 vom Kopf 24 der zweiten Destillationskolonne 23 in die Methacroleinaufbereitungsanlage 110 geleitet werden kann. Die dritte Destillationskolonnenabführleitungsanordnung 26 verbindet den Kopf 24 der zweiten Destillationskolonne 23 mit dem ersten Kondensator 9 fluidisch, wobei drittes Destillationsstoffgemisches durch die dritte Destillationskolonnenabführleitungsanordnung 26 vom Kopf 24 der zweiten Destillationskolonne 23 in den ersten Kondensator 9 geleitet werden kann.

Die dritte Destillationskolonnenabführleitungsanordnung 26 ist an einen eigenen Eingang des ersten Kondensators 9 angeschlossen.

An den Sumpf 25 der zweiten Destillationskolonne 23 ist eine vierte Destillationskolonnenabführleitungsanordnung 27 fluidisch angeschlossen, durch welche viertes Destillationsstoffgemisch aus der zweiten Destillationskolonne 23 abgeführt werden kann.

Die Herstellanlage 100 weist eine Membrananlage 28 auf, welche mit dem Sumpf 13 der ersten Destillationskolonne 11 fluidisch verbunden ist. Die Membrananlage 28 ist durch die zweite Destillationskolonnenabführleitungsanordnung 15 fluidisch mit dem Sumpf 13 der ersten Destillationskolonne 11 verbunden, wobei zweites Destillationsstoffgemisch durch die zweite Destillationskolonnenabführleitungsanordnung 15 vom Sumpf 13 der ersten Destillationskolonne 11 in die Membrananlage 28 geleitet werden kann.

Durch die zweite Destillationskolonnenabführleitungsanordnung 15 kann zweites Trennstoffgemisch auch aus der Herstellanlage 100 abgeführt werden.

Die zweite Destillationskolonnenabführleitungsanordnung 15 der in Figur 1 gezeigten Ausführungsform weist einen ersten Abschnitt 29, einen zweiten Abschnitt 30, einen dritten Abschnitt 31 und einen vierten Abschnitt 32 auf. Der erste Abschnitt 29 ist einerseits mit dem Sumpf 13 der ersten Destillationskolonne 11 fluidisch verbunden und andererseits mit dem zweiten Abschnitt 30, dem dritten Abschnitt 31 und dem vierten Abschnitt 32 fluidisch verbunden.

Der zweite Abschnitt 30 ist fluidisch mit dem Reaktor 1 verbunden, wodurch zweites Destillationsstoffgemisch vom Sumpf 13 der ersten Destillationskolonne 11 durch den ersten und den zweiten Abschnitt 29, 30 der zweiten Destillationskolonnenabführleitungsanordnung 15 vom Sumpf 13 der ersten Destillationskolonne 11 in den Reaktor 1 geleitet werden kann. Über den ersten und den dritten Abschnitt 29, 31 der zweiten Destillationskolonnenabführleitungsanordnung 15 kann zweites Destillationsstoffgemisch aus der Herstellanlage 100 abgeführt werden.

Der vierte Abschnitt 32 ist fluidisch mit der Membrananlage 28 verbunden, wodurch zweites Destillationsstoffgemisch durch den ersten und den vierten Abschnitt 29, 32 der zweiten Destillationskolonnenabführleitungsanordnung 15 vom Sumpf 13 der ersten Destillationskolonne 11 in die Membrananlage 28 geleitet werden kann.

Mit der Membrananlage 28 kann wenigstens ein Teil des Katalysators, welcher in dem der Membrananlage 28 zugeleiteten zweiten Destillationsstoffgemisch enthalten ist, zurückgehalten werden. Die Membrananlage 28 weist einen ersten Ausgang 33 zum Abführen von Retentatstoffgemisch, welches den zurückgehaltenen Katalysator enthält, auf sowie einen zweiten Ausgang 34 zum Abführen von Permeatstoffgemisch. Der erste Ausgang 33 ist mit einer ersten Membrananlagenabführleitungsanordnung 35 fluidisch verbunden, durch welche Retentatstoffgemisch über den ersten Ausgang 33 der Membrananlage 28 aus der Membrananlage 28 abgeführt werden kann.

In dem in Figur 1 gezeigten Ausführungsbeispiel weist die erste Membrananlagenabführleitungsanordnung 35 einen ersten Abschnitt 36, einen zweiten Abschnitt 37 und einen dritten Abschnitt 38 auf. Der erste Abschnitt 36 ist einerseits mit dem ersten Ausgang 33 der Membrananlage 28 fluidisch verbunden und andererseits mit dem zweiten Abschnitt 37 und dem dritten Abschnitt 38 der ersten Membrananlagenabführleitungsanordnung 35. Der zweite Abschnitt 37 der ersten Membrananlagenabführleitungsanordnung 35 ist fluidisch mit dem zweiten Abschnitt 30 der zweiten Destillationskolonnenabführleitungsanordnung 15 verbunden, wodurch Retentatstoffgemisch von der Membrananlage 28 über deren ersten Ausgang 33, den ersten und zweiten Abschnitt 36, 37 der ersten Membrananlagenabführleitungsanordnung 35 in den zweiten Abschnitt 30 der zweiten Destillationskolonnenabführleitungsanordnung 15 geleitet werden kann und durch diesen weiter in den Reaktor 1.

Durch den ersten und den dritten Abschnitt 36, 38 der ersten Membrananlagenabführleitungsanordnung 35 kann Retentatstoffgemisch aus der Herstellanlage 100 abgeführt werden.

Mit dem zweiten Ausgang 34 der Membrananlage 28 ist eine zweite Membrananlagenabführleitungsanordnung 39 fluidisch verbunden, wobei Permeatstoffgemisch vom zweiten Ausgang 34 der Membrananlage 28 durch die zweite Membrananlagenabführleitungsanordnung 39 aus der Herstellanlage 100 ausgeschleust werden kann.

In der in Figur 1 gezeigten Ausführungsform der vorliegenden Erfindung ist eine Formaldehydquelle 40 über eine Formaldehydzuleitungsanordnung 41 fluidisch mit der zweiten Destillationskolonnenabführleitungsanordnung 15 verbunden, wodurch Formaldehyd durch die Formaldehydzuleitungsanordnung 41 in die zweite Destillationskolonnenabführleitungsanordnung 15 geleitet werden kann und durch diese weiter in den Reaktor 1. Eine Propionaldehydquelle 42 ist über eine Propionaldehydzuleitungsanordnung 43 mit der zweiten Destillationskolonnenabführleitungsanordnung 15 fluidisch verbunden, wodurch Propionaldehyd von der Propionaldehydquelle 42 durch die Propionaldehydzuleitungsanordnung 43 in die zweite Destillationskolonnenabführleitungsanordnung 15 geleitet werden kann und durch diese weiter in den Reaktor 1. Eine Basenquelle 44 ist über eine Basenzuleitungsanordnung 45 fluidisch mit der zweiten Destillationskolonnenabführleitungsanordnung 15 verbunden, wodurch eine Base oder Basen durch die Basenzuleitungsanordnung 45 in die zweite Destillationskolonnenabführleitungsanordnung 15 geleitet werden kann bzw. können und durch diese weiter in den Reaktor 1. Eine Säurenquelle 46 ist über eine Säurenzuleitungsanordnung 47 fluidisch mit der zweiten Destillationskolonnenabführleitungsanordnung 15 fluidisch verbunden, wodurch Säure oder Säuren durch die Säurenzuleitungsanordnung 47 in die zweite Destillationskolonnenabführleitungsanordnung 15 geleitet werden kann bzw. können und durch diese weiter in den Reaktor 1.

Mit der Herstellanlage 100 kann beispielsweise das nachfolgend beschriebene Verfahren zum Herstellen von Methacrolein aus Formaldehyd und Propionaldehyd in Gegenwart von Wasser und einem homogenen Katalysator, welcher wenigstens auf einer Säure und einer Base basiert, durchgeführt werden.

Durch den zweiten Abschnitt 30 der zweiten Destillationskolonnenabführleitungsanordnung 15 werden Formaldehyd, Propionaldehyd, Wasser und homogener Katalysator in den Reaktor 1 eingeleitet. Dabei wird frisches Formaldehyd aus der Formaldehydquelle 40 über die Formaldehydzuleitungsanordnung 41 in den zweiten Abschnitt 30 der zweiten Destillationskolonnenabführleitungsanordnung 15 eingeleitet, frisches Propionaldehyd aus der Propionaldehydquelle 42 über die Propionaldehydzuleitungsanordnung 43, eine oder mehrere frische Basen von der Basenquelle 44 über die Basenzuleitungsanordnung 44 und eine oder mehrere frische Säuren von der Säurenquelle 46 über die Säurenzuleitungsanordnung 47. Das Formaldehyd, die Säure bzw. die Säuren sowie die Base bzw. die Basen liegen jeweils in einer wässrigen Lösung vor, d. h. diese wässrigen Lösungen bringen Wasser mit. Die Säure bzw. die Säuren sind in Verbindung mit der Base bzw. den Basen der homogene Katalysator.

Geeignete wässrige Formaldehydlösungen sind beispielsweise solche mit einem auf die Gesamtmasse der Formaldehydlösung bezogenen Gehalt von 30 bis 55 Gew.-% Formaldehyd mit einem vorzugsweise niedrigen Methanolgehalt von beispielsweise 0,3 bis 10 Gew.-% bezogen auf die Gesamtmasse der Formaldehydlösung.

Propionaldehyd ist hochkonzentriert verfügbar. So ist Propionaldehydeinsatzstoffgemisch beispielsweise mit einem Restwassergehalt von 0,1 bis 2,5 Gew.-% bezogen auf die Gesamtmasse des Propionaldehyeinsatzstoffgemisches und einem Propionsäuregehalt von 0,01 bis 1 Gew.-% bezogen auf die Gesamtmasse des Propionaldehydeinsatzstoffgemisches verfügbar.

Bevorzugt wird ein leichter Überschuss von Formaldehyd gegenüber Propionaldehyd im Reaktoreingang. Besonders bevorzugt wird ein molares Verhältnis von Propionaldehyd zu Formaldehyd im Reaktoreingang im Bereich von 0,90 bis 0,99 gewählt, ganz besonders bevorzugt 0,95 bis 0,98. Hiermit kann einerseits ein guter Umsatz bei geringem Formaldehydverbrauch erreicht werden. Andererseits wird dabei aber auch noch der Katalysator geschont. Denn zu hohe Formaldehydgehalte fördern die Umwandlung von Dimethylamin zu Trimethylamin.

Geeignete Säuren sind insbesondere anorganische Säuren, organische Monocarbonsäuren, organische Dicarbonsäuren und organische Polycarbonsäuren. Andere organische Säuren sind prinzipiell auch verwendbar, kommen aber meist aus Preisgründen nicht zum Einsatz. Bevorzugt werden organische Monocarbonsäuren eingesetzt.

Als anorganische Säuren können beispielsweise Schwefelsäure und/oder Phosphorsäure eingesetzt werden.

Von den organischen Monocarbonsäuren werden aliphatische organische Monocarbonsäuren bevorzugt. Von den aliphatischen Monocarbonsäuren werden solche mit zwei bis zehn Kohlenstoffatomen bevorzugt, besonders solche mit zwei, drei oder vier Kohlenstoffatomen.

Von den aliphatischen Di- und Polycarbonsäuren werden solche mit zwei bis zehn Kohlenstoffatomen bevorzugt, besonders solche mit zwei, vier, fünf oder sechs Kohlenstoffatomen. Die organischen Dicarbonsäuren und die organischen Polycarbonsäuren können aromatische, araliphatische und aliphatische Carbonsäuren sein, wobei aliphatische Di- und Polycarbonsäuren bevorzugt werden.

Beispielsweise können Essigsäure, Propionsäure, Methoxyessigsäure, n-Buttersäure, isoButtersäure, Oxalsäure, Bernsteinsäure, Weinsäure, Glutarsäure, Adipinsäure, Maleinsäure oder Fumarsäure verwendet werden, wobei Essigsäure bevorzugt wird.

Es können auch Gemische von zwei oder mehr Säuren eingesetzt werden.

Geeignete Basen sind insbesondere organische Basen, wobei Amine bevorzugt werden. Von den Aminen werden sekundäre Amine bevorzugt, besonders solche der Formel R¹R²NH, bei denen R¹ und R²
- gleich oder verschieden sind und
- jeweils bezeichnen können:
   ▪ einen Alkylrest mit einem bis zehn Kohlenstoffatomen, bevorzugt einem bis acht Kohlenstoffatomen, besonders bevorzugt einem bis vier Kohlenstoffatomen, wobei die Kohlenstoffatome auch durch Ether-, Hydroxy-, sekundäre oder tertiäre Aminogruppen substituiert sein können, bevorzugt durch eine oder zwei dieser Gruppen
   ▪ einen Aralkylrest mit sieben bis zwölf Kohlenstoffatomen
   ▪ einen Cycloalkylrest mit fünf bis sieben Kohlenstoffatomen
   ▪ mit dem benachbarten Stickstoff Glieder eines heterocyclischen Ringes, bevorzugt eines fünf- bis siebengliedrigen Ringes, der noch ein weiteres Stickstoffatom und/oder ein Sauerstoffatom enthalten kann und durch Hydroxyalkyl- oder Alkylgruppen mit einem bis vier Kohlenstoffatomen substituiert sein kann

Es können eine oder mehrere Basen eingesetzt werden.

Als Amine können beispielsweise eingesetzt werden: Dimethylamin, Diethylamin, Methylethylamin, Methylpropylamin, Dipropylamin, Dibutylamin, Di-isopropylamin, Di-iso-butylamin, Methyl-isopropylamin, Methyl-iso-butylamin, Methyl- sek.-butylamin, Methyl-(2-methylpentyl)-amin, Methyl-(2-ethylhexyl)-amin, Pyrrolidin, Piperidin, Morpholin, N-Methylpiperazin, N-Hydroxyethyl-piperazin, Piperazin, Hexamethylenimin, Diethanolamin, Methylethanolamin, Methylcyclohexylamin, Methylcyclopentalamin oder Dicyclohexylamin

Es können auch Gemische von zwei oder mehr Basen eingesetzt werden.

Wird ein Gemisch von Aminen eingesetzt, werden die Amine bevorzugt so ausgewählt, dass mindestens eines der eingesetzten Amine keine Hydroxygruppe aufweist. Besonders bevorzugt beträgt der Anteil an Aminen mit mindestens einer Hydroxygruppe im Reaktor höchstens 50 Gew.-%, vorzugsweise höchstens 30 Gew.-%, und besonders bevorzugt höchstens 10 Gew.-%, bezogen auf das Gewicht der eingesetzten Amine.

Der Anteil der Säure bzw. Säuren (insgesamt) bezogen auf Propionaldehyd im Reaktor liegt bevorzugt im Bereich von 0,1 bis 20 Mol-%, besonders bevorzugt im Bereich von 0,5 bis 10 Mol-% und ganz besonders bevorzugt im Bereich von 1 bis 5 Mol-%. Bei zu geringer Säuremenge - und damit zu geringer Katalysatormenge - im Reaktor müsste der Reaktor recht groß gebaut und recht heiß betrieben werden. Bei zu hoher Säuremenge - und damit zu hoher Katalysatormenge - würde der Reaktor recht heiß werden.

Es empfiehlt sich, etwas mehr Säure im Vergleich zur Base im Reaktor zu haben. Dies ist vorteilhaft, da die Mannich-Reaktion Protonen benötigt. Außerdem können Säuren Basen binden. Wenn beispielsweise als Säure Essigsäure gewählt wird und als Base Dimethylamin, bindet die Essigsäure das leicht flüchtige Dimethylamin. Hierdurch wird dem Fall entgegengewirkt, dass das Dimethylamin als leicht flüchtige Komponente in den Kopf der ersten Destillationskolonne 11 gelangt.

Wenn beispielsweise als Säure Essigsäure gewählt wird und als Base Dimethylamin, ist ein molares Verhältnis von Essigsäure zu Dimethylamin im Bereich von 2,0 bis 1,1 am Reaktoreingang gut geeignet, wobei ein molares Verhältnis von Essigsäure zu Dimethylamin von 1,1 besonders bevorzugt wird.

Der Anteil der Base bzw. Basen (insgesamt) bezogen auf Propionaldehyd im Reaktoreingang liegt bevorzugt im Bereich von 0,1 bis 20 Mol-% bezogen auf Mole Propionaldehyd am Eingang des Reaktors, besonders bevorzugt im Bereich von 0,5 bis 15 Mol-% bezogen auf Mole Propionaldehyd am Eingang des Reaktors und ganz besonders bevorzugt im Bereich von 1 bis 10 Mol-% bezogen auf Mole Propionaldehyd am Eingang des Reaktors. Wird zu wenig Base bzw. Basen eingesetzt, ist die Reaktion unselektiver und der Reaktor muss bei höheren Temperaturen betrieben werden. Wird zu viel Base bzw. Basen eingesetzt, wird die Durchführung der Reaktion zunehmend unwirtschaftlich und das Abwasser fällt noch stärker belastet aus.

Wird als Base beispielsweise Dimethylamin eingesetzt, liegt das molare Verhältnis von Dimethylamin zu Propionaldehyd im Reaktoreingang bevorzugt im Bereich von 0,08 bis 0,12, besonders bevorzugt beträgt es 0,08 bis 0,1.

Bevorzugt ist ein stöchiometrisches Verhältnis von Säure zu Base von größer eins, um zum Spalten der Mannich-Base ausreichend Acedität zu haben und, insbesondere wenn eine leicht flüchtige Base eingesetzt wird, die Base gut zu binden können, um sie gut in den Sumpf 13 der ersten Destillationskolonne zu überführen zu können und von dort aus abführen zu können.

Im Reaktor 1 findet in der flüssigen Phase eine Mannich-Reaktion statt, bei welcher Formaldehyd und Propionaldehyd in Gegenwart von Wasser und in Gegenwart des homogenen Katalysators zu Methacrolein umgesetzt werden.

Die Reaktionstemperatur am Auslass der Reaktionszone des Reaktors 1 wird so gewählt, dass sie bevorzugt im Bereich von 100 °C bis 210 °C liegt, besonders bevorzugt im Bereich von 110 °C bis 200 °C, ganz besonders bevorzugt im Bereich von 120 °C bis 190 °C und ganz speziell bevorzugt im Bereich von 130 °C bis 180 °C. Hiermit können ein hoher Umsatz und eine gute Ausbeute erreicht werden.

Der Druck in dem Reaktor 1 wird wenigstens so hoch gewählt, dass das Reaktionsgemisch im Reaktor 1 flüssig bleibt. Unter dieser Prämisse wird ein passender Druck im Reaktor 1 eingestellt, der bevorzugt im Bereich von 15 bis 100 bar liegt, besonders bevorzugt im Bereich von 18 bis 80 bar, ganz besonders bevorzugt im Bereich von 22 bis 50 bar und ganz speziell bevorzugt im Bereich von 25 bis 40 bar. Bei den Drücken handelt es sich um absolute Drücke.

Auch nachdem das flüssige Reaktionsgemisch die Reaktionszone des Reaktors 1 verlassen hat, können die Mannich-Reaktion und auch andere Folgereaktionen, wie beispielsweise Umsetzung von Methacrolein zu Di-Methacrolein oder die Methacrolein-Oligomerisierung, noch in erheblichem Maß ablaufen, insbesondere wenn das Reaktionsgemisch weiter unter dem Druck steht, der in der Reaktionszone herrscht, und im Wesentlichen weiterhin die Temperatur hat, die es beim Austreten aus der Reaktionszone hat. Als Reaktionsverweilzeit wird vorliegend die durchschnittliche Zeit verstanden, die zwischen dem Eintreten der Edukte in die Reaktionszone des Reaktors 1 und dem Beginn des Entspannens des Reaktionsgemisches im Entspannungsventil 3 vergeht. Die Verweilzeit in der Reaktionszone liegt vorzugsweise im Bereich von 0,001 Minuten bis 25 Minuten, besonders bevorzugt im Bereich von 0,001 Minuten bis 10 Minuten, ganz besonders bevorzugt im Bereich von 0,1 Sekunden bis 300 Sekunden, ganz speziell bevorzugt im Bereich von 1 Sekunde bis 50 Sekunden, noch weiter bevorzugt im Bereich von 5 bis 20 Sekunden. Hiermit können bei guter Ausbeute Neben- und Folgereaktionen gut begrenzt werden.

Das Reaktionsgemisch enthält Methacrolein und die Begleitkomponenten Wasser, nicht umgesetztes Formaldehyd, nicht umgesetztes Propionaldehyd und Katalysator. Es kann außerdem eine oder mehrere der folgenden weiteren Begleitkomponenten enthalten:
- Methanol, wenn Methanol in der frischen wässrigen Formaldehydlösung enthalten war
- Di-Methacrolein
- Trimethylamin, wenn Dimethylamin als Base eingesetzt wird
- Hochsieder, insbesondere hochsiedende Adolisierungsprodukte
- Oligomere, insbesondere Oligomere des Methacroleins
- Stabilisator (z. B. Tempol)
- Dimere des Proprionaldehyds

Über die Reaktorabführleitungsanordnung 2 wird das Reaktionsgemisch zum Entspannungsventil 3 geleitet und mithilfe des Entspannungsventils 3 auf einen gewünschten Druck entspannt, beispielsweise auf einen Druck im Bereich von 500 mbar (absolut) bis Normaldruck. Durch das Entspannen des Druckes geht wenigstens ein Teil des Reaktionsgemisches von der flüssigen Phase in die gasförmige Phase über, wodurch ein erstes Entspannungsstoffgemisch entsteht, nämlich die durch die Entspannung in die Gasphase übergehende Fraktion des Reaktionsgemisches. Die nach der Entspannung in flüssiger Phase verbleibende Fraktion des Reaktionsgemisches ist ein zweites Entspannungsstoffgemisch.

Da das Methacrolein-Wasser-Azeotrop einen niedrigeren Siedepunkt hat als Wasser und daher leichter verdampft als Wasser, enthält das erste Entspannungsstoffgemisch einen höheren Gewichtsanteil an Methacrolein als das zweite Entspannungsstoffgemisch. Dabei können durchaus mehr als 90 Gew.-% des im Reaktionsgemisch insgesamt enthaltenen Metharcoleins in das erste Entspannungsstoffgemisch gelangen. D. h. das Entspannen mithilfe des Entspannungsventils 3 ist bereits ein erster Schritt zum Aufreinigen von Methacrolein, wenngleich das Entspannungsventil 3 nicht zu der erfindungsgemäßen Methacroleinaufarbeitungsanlage 110 gehört.

Der Druckabbau und der Übergang eines Teiles des Reaktionsgemisches in die gasförmige Phase führen zu einer Verringerung der Temperatur des in die Gasphase übergehenden Teiles des Reaktionsgemisches (erstes Entspannungsstoffgemisch) und des in flüssiger Phase verbleibenden Teiles des Reaktionsgemisches (zweites Entspannungsstoffgemisch). Für diesen Abkühlungseffekt ist folglich keine anderweitige Kühlung erforderlich.

Vorzugsweise wird das Verfahren so betrieben, dass das erste und zweite Entspannungsstoffgemisch nach der Entspannung eine Temperatur im Bereich von ca. 60 bis 120 °C haben, vorzugsweise eine Temperatur im Bereich von ca. 70 bis 110 °C und besonders bevorzugt eine Temperatur im Bereich von ca. 80 bis 90 °C. Es hat sich herausgestellt, dass in diesen Temperaturbereichen das Ausmaß der unerwünschten Neben- und Folgereaktionen, die stattfinden können, weniger kritisch ist als gedacht. Insbesondere können das erste und das zweite Entspannungsstoffgemisch einige Minuten in den angegebenen Temperaturbereichen bleiben, vorzugsweise 0,1 bis 15 Minuten, besonders bevorzugt 1 bis 5 Minuten, noch weiter bevorzugt 0,5 bis 3 Minuten. Dies sind Verweilzeiten, die im Hinblick auf unerwünschte Neben- und Folgereaktionen gut akzeptabel sind.

Vorzugsweise ist die Verweilzeit des ersten Entspannungsstoffgemisches im Entspannungsbehälter 5 geringer als die des zweiten Entspannungsstoffgemisches.

Wenn das dem Entspannungsventil 3 zugeführte Reaktionsgemisch beispielsweise eine Temperatur von 165 °C und einen Druck von 35 bar (absolut) hat und die Zusammensetzung bezogen auf ihre Gesamtmasse 63,51 Gew.-% Wasser und 29,04 Gew.-% Methacrolein enthält, kann eine schnelle Entspannung auf 0,85 bar (absolut) zu einer Temperatur des ersten und zweitens Entspannungsstoffgemisches von 78,9 °C führen (vergleiche erfindungsgemäßes Versuchsbeispiel).

Durch die Entspannungsventilabführleitungsanordnung 4 werden das erste Entspannungsstoffgemisch und das zweite Entspannungsstoffgemisch in den Entspannungsbehälter 5 geleitet. In dem Entspannungsbehälter 5 werden das erste Entspannungsstoffgemisch und das zweite Entspannungsstoffgemisch voneinander getrennt.

Das erste Entspannungsstoffgemisch wird über den ersten Ausgang 6 des Entspannungsbehälters 5 und die erste Entspannungsbehälterabführleitungsanordnung 8 in den ersten Kondensator 9 geleitet. Das zweite Entspannungsstoffgemisch wird über den zweiten Ausgang 7 des Entspannungsbehälters 5 und die zweite Entspannungsbehälterabführleitungsanordnung 10 in die erste Destillationskolonne 11 geleitet.

Vorzugsweise wird der Entspannungsbehälter 5 so betrieben, dass in ihm ein guter Füllstand zweiten Entspannungsstoffgemisches vorhanden ist, wobei die Verweilzeit des zweiten Entspannungsstoffgemisches im Entspannungsbehälter 5 deutlich höher ist als die Verweilzeit des ersten Entspannungsstoffgemisches.

Im ersten Kondensator 9 wird das erste Entspannungsstoffgemisch kondensiert.

In der ersten Destillationskolonne 11 wird das zweite Entspannungsstoffgemisch in ein erstes Destillationsstoffgemisch und ein zweites Destillationsstoffgemisch getrennt, wobei das erste Destillationsstoffgemisch als Gasphase am Kopf 12 der ersten Destillationskolonne 11 vorliegt und das zweite Destillationsstoffgemisch im Sumpf 13 der ersten Destillationskolonne 11 als flüssige Phase. Das erste Destillationsstoffgemisch hat einen höheren Gewichtsanteil an Methacrolein als das zweite Destillationsstoffgemisch. Das erste Destillationsstoffgemisch enthält außerdem nicht umgesetztes Propionaldehyd und nicht umgesetztes Formaldehyd. Das zweite Destillationsstoffgemisch hat einen höheren Gewichtsanteil an Wasser als das erste Destillationsstoffgemisch. Zudem enthält das zweite Destillationsstoffgemisch gegenüber dem ersten Destillationsstoffgemisch wenigstens den größeren Teil des durch das zweite Entspannungsstoffgemisch zugeleiteten Katalysators. Außerdem enthält das zweite Destillationsstoffgemisch nicht umgesetztes Formaldehyd.

Es ist möglich, den Prozess in der ersten Destillationskolonne 11 so zu gestalten, dass nahezu aller des durch das zweite Entspannungsstoffgemisch zugeleiteten Katalysators im Sumpf 13 der ersten Destillationskolonne 11 vorliegt.

Die erste Destillationskolonne 11 kann beispielsweise bei Normaldruck so betrieben werden, dass in ihrem Sumpf 13 eine Temperatur von etwas über 100 °C herrscht. Die Erhöhung etwas über 100 °C (für Normaldruck) hinaus ist durch den in dem Sumpf 13 vorhanden Katalysatorgehalt bedingt, durch welchen sich die Siedetemperatur zweiten Destillationsstoffgemisches entsprechend erhöht.

Die Temperatur am Kopf 12 der ersten Destillationskolonne 11 kann bei Normaldruck im Bereich von 68 bis 98 °C gewählt werden, vorzugsweise im Bereich von 80 bis 90 °C. Dadurch gelangt mehr Wasser in den Kopf 12 als bei niedrigeren Temperaturen am Kopf 12.

Über die erste Destillationskolonnenabführleitungsanordnung 14 wird das erste Destillationsstoffgemisch in den ersten Kondensator 9 geleitet und dort ebenfalls kondensiert. Das Kondensat des ersten Kondensators 9 enthält eine organische Phase (erstes Trennstoffgemisch), welche Methacrolein enthält, und eine wässrige Phase (zweites Trennstoffgemisch).

Das Kondensat des ersten Kondensators 9 wird über dessen Kondensatausgang 16 und die Kondensatorabführleitungsanordnung 17 in den ersten Phasentrenner 18 geleitet. Dort werden die organische und die wässrige Phase, also erstes und zweites Trennstoffgemisch, voneinander getrennt. Die organische Phase, d. h. das erste Trennstoffgemisch, wird über den ersten Ausgang 19 des ersten Phasentrenners 18 und die erste Phasentrennerabführleitungsanordnung 21 abgeführt, beispielsweise in einen Tank oder eine weiterverarbeitende Anlage, wie zum Beispiel eine Anlage zum Herstellen von Methylmethacrylat. Die wässrige Phase, d. h. das zweite Trennstoffgemisch, wird über den zweiten Ausgang 20 des ersten Phasentrenners 18 und die zweite Phasentrennerabführleitungsanordnung 22 in die zweite Destillationskolonne 23 geleitet. Der Wasseranteil im zweiten Trennstoffgemisch kann bezogen auf die Gesamtmasse des zweiten Trennstoffgemisches durchaus bei 75 Gew.-% oder höher liegen. Der Gehalt an Methacrolein im zweiten Trennstoffgemisch kann bezogen auf die Gesamtmasse des zweiten Tennstoffgemisches durchaus im Bereich von 2 bis 10 Gew.-% liegen. Der Gehalt an Methanol im zweiten Trennstoffgemisch kann bezogen auf die Gesamtmasse des zweiten Tennstoffgemisches durchaus im Bereich von 1 bis 10 Gew.-% liegen. Das zweite Trennstoffgemisch kann auch Formaldehyd enthalten. Es versteht sich von selbst, dass die Gesamtheit aller Bestandteile des zweiten Trennstoffgemisches zusammen 100 Gew.-% der Gesamtmasse des zweiten Trennstoffgemisches ausmacht.

Dadurch, dass das zweite Trennstoffgemisch in die zweite Destillationskolonne 23 und nicht in die erste Destillationskolonne 11 geleitet wird, gelangt weniger Wasser in die erste Destillationskolonne 11. Dies hat zur Folge, dass die Konzentration des Katalysators im Sumpf 13 der ersten Destillationskolonne 11 höher ist als wenn das zweite Trennstoffgemisch in die erste Destillationskolonne 11 eingeleitet werden würde. Da die in den Reaktor 1 zurückführbare Wassermenge begrenzt ist, ist auch die Menge des in den Reaktor 1 zurückführbaren zweiten Destillationsstoffgemisches wegen der hierin enthaltenen Wassermenge begrenzt. Da das zweite Destillationsstoffgemisches gemäß der vorliegenden Erfindung einen höheren Anteil an Katalysator enthält, kann ein größerer Teil dieses Katalysators in den Reaktor 1 zurückgeführt werden. Dementsprechend kann frischer Katalysator eingespart werden.

Da der ersten Destillationskolonne 11 weniger Wasser zugeführt wird, fällt auch eine geringere Menge zweiten Destillationsstoffgemisches an. Somit muss, falls zweites Destillationsstoffgemisches entsorgt werden soll, eine geringere Menge zweiten Destillationsstoffgemisches entsorgt werden.

Das zweite Trennstoffgemisch wird in der zweiten Destillationskolonne 23 in ein drittes Destillationsstoffgemisch und ein viertes Destillationsstoffgemisch getrennt, wobei das dritte Destillationsstoffgemisches als Gasphase am Kopf 24 der zweiten Destillationskolonne 23 vorliegt und Methacrolein enthält und das vierte Destillationsstoffgemisches im Sumpf 25 der zweiten Destillationskolonne 23 als flüssige Phase vorliegt und Wasser enthält. Da der Katalysator zum größten Teil in den Sumpf 13 der ersten Destillationskolonne 11 gelangt, gelangt entsprechend wenig Katalysator in den Sumpf 25 der zweiten Destillationskolonne 23. Dementsprechend ist das vierte Destillationsstoffgemisch von seinen Umwelteigenschaften her weniger problematisch als das zweite Destillationsstoffgemisch. Da es möglich ist, die erste Destillationskolonne 11 so zu betreiben, dass sich fast aller des in dem zweiten Entspannungsstoffgemisch enthaltenen Katalysators im Sumpf 13 der ersten Destillationskolonne 11 sammelt, und da es ergänzend außerdem möglich ist, mit einer guten Effektivität Tropfen und Tröpfchen zweiten Entspannungsstoffgemisches im Entspannungsbehälter 5 daran zu hindern, diesen über dessen ersten Ausgang 6 zu verlassen, ist es möglich, ein viertes Destillationsstoffgemisches zu erhalten, in welchem nur wenig oder kaum Katalysator enthalten ist. Somit kann ein viertes Destillationsstoffgemisch erhalten werden, welches im Hinblick auf den Katalysator wenig oder praktisch kaum umweltbelastend ist und diesbezüglich einfach entsorgt werden kann, beispielsweise indem es in eine kommunale Kläranlage abgegeben wird.

Die zweite Destillationskolonne 23 kann beispielsweise bei Normaldruck so betrieben, dass in ihrem Sumpf 25 eine Temperatur im Bereich von ca. 100 bis 102 °C herrscht, um zu begünstigen, dass die Leichtsieder, wie beispielsweise Methanol und Methacrolein, in den Kopf 24 der zweiten Destillationskolonne gelangen.

Die Temperatur am Kopf 24 der zweiten Destillationskolonne kann bei Normaldruck im Bereich von 65 bis 99 °C gewählt werden, vorzugsweise im Bereich von 70 bis 95 °C. Hierdurch wird begünstigt, dass das Abwasser gut an Leichtsiedern abgereichert wird.

Beispielsweise kann ein drittes Destillationsstoffgemisch erhalten werden, welches bezogen auf seine Gesamtmasse mehr als 30 Gew.-% Methacrolein und mehr als 40 Gew.-% Wasser enthält. Als weitere Komponenten kann es vor allem Methanol enthalten.

Dabei kann beispielsweise kann ein viertes Destillationsstoffgemisch erhalten werden, welches bezogen auf seine Gesamtmasse mehr als 98 Gew.-% Wasser und weniger als 1 Gew.-% Methanol enthält und weitestgehend von Methacrolein befreit ist.

Das dritte Destillationsstoffgemisch wird durch die dritte Destillationskolonnenabführleitungsanordnung 26 vom Kopf 24 der zweiten Destillationskolonne 23 in den ersten Kondensator 9 geleitet und dort kondensiert.

Das vierte Destillationsstoffgemisch wird über die vierte Destillationskolonnenabführleitungsanordnung 27 vom Sumpf 25 der zweiten Destillationskolonne 23 aus der Herstellanlage ausgeschleust. Es kann in einem Tank gesammelt oder einer weiterverarbeitenden Anlage zugeführt werden, beispielsweise einer Herstellstellanlage zum Herstellen von Methylmethacrylat, wobei das in dem vierten Destillationsstoffgemisch enthaltene Methacrolein ein Edukt zum Herstellen von Methylmethacrylat ist.

Das zweite Destillationsstoffgemisch wird über den ersten Anschnitt der zweiten Destillationskolonnenabführleitungsanordnung 15 geleitet. Anschließend wird
- der Teil des zweiten Destillationstoffgemisches, der zum Reaktor 1 geleitet werden soll, in den zweiten Abschnitt 30 der zweiten Destillationskolonnenabführleitungsanordnung 15 und somit zum Reaktor 1 geleitet,
- der Teil des zweiten Destillationstoffgemisches, der aus der Methacroleinaufbereitanlage 110 herausgeleitet werden soll, in den dritten Abschnitt 31 der zweiten Destillationskolonnenabführleitungsanordnung 15 geleitet, und
- der Teil des zweiten Destillationstoffgemisches, der in die Membrananlage 28 geleitet werden soll, in den vierten Anschnitt 32 der zweiten Destillationskolonnenabführleitungsanordnung 15 geleitet.

Wenn der dritte Abschnitt 31 der zweiten Destillationskolonnenabführleitungsanordnung 15 nicht nur aus der Methacroleinaufbreitungsanlage 110, sondern auch aus der Herstellanlage 100 insgesamt herausführt, wie dies bei der ersten Ausführungsform der Erfindung der Fall ist, ist der Teil des zweiten Destillationstoffgemisches, der in den dritten Abschnitt 31 der zweiten Destillationskolonnenabführleitungsanordnung 15 geleitet wird, der Teil des zweiten Destillationstoffgemisches, der aus der Herstellanlage 100 ingesamt herausgeleitet werden soll.

In der Membrananlage 28 wird wenigstens ein Teil des im zweiten Destillationsstoffgemisch enthaltenen Katalysators zurückgehalten (Retentatstoffgemisch). Dementsprechend hat das Retentatstoffgemisch eine höhere Konzentration an Katalysator als das zweite Destillationsstoffgemisch. Das Permeatstoffgemisch der Membrananlage 28 hat einen entsprechend geringeren Anteil an Katalysator. Dementsprechend ist das Permeatstoffgemisch, was seinen Gehalt an Katalysator angeht, weniger umweltbelastend als das Retentatstoffgemisch. Mit einer entsprechend effektiven Membrananlage ist es möglich, ein Permeatstoffgemisch zu erhalten, welches eine so geringe Konzentration an Katalysator hat, dass das Permeatstoffgemisch im Hinblick auf seine Konzentration an Katalysator problemlos entsorgt werden kann, beispielsweise in dem es in eine kommunale Kläranlage abgegeben wird.

Als Membrananlage wird eine Umkehrosmosemembrananlage bevorzugt.

Das Retentatstoffgemisch wird über den ersten Ausgang 33 der Membrananlage 28 in den ersten Abschnitt 36 der ersten Membrananlagenabführleitungsanordnung 35 geleitet. Der Teil des Retentatstoffgemisches, der in den Reaktor 1 geleitet werden soll, wird durch den zweiten Abschnitt 37 der ersten Membrananlagenabführleitungsanordnung 35 in den zweiten Abschnitt 30 der zweiten Destillationskolonnenabführleitungsanordnung 15 geleitet. Der Teil des Retentatstoffgemisches, welcher aus der Herstellanlage 100 abgeführt werden soll, wird durch den dritten Abschnitt 38 der ersten Membrananlagenabführleitungsanordnung 35 aus der Herstellanlage 100 herausgeleitet.

Permeatstoffgemisch wird über den zweiten Ausgang 34 der Membrananlage 28 und die zweite Membrananlagenabführleitungsanordnung 39 aus der Herstellanlage 100 abgeführt.

Wenn das Permeatstoffgemisch ausreichend wenig Katalysator enthält, kann es, jedenfalls im Hinblick auf seinen Katalysatorgehalt, in eine öffentliche Kläranlage zur Entsorgung abgegeben werden.

Geht es darum, im zweiten Destillationsstoffgemisch enthaltenen Katalysator, Trimethylamin und/oder unerwünschte Hochsieder zu entsorgen, ist die Entsorgung von Retentatstoffgemisch insofern günstiger als die direkte Entsorgung von zweitem Destillationsstoffgemisch über den dritten Abschnitt 31 der zweiten Destillationskolonnenabführleitungsanordnung 15, als das Retentatstoffgemisch bezogen auf die gleiche Menge an Katalysator, Trimethylamin und/oder unerwünschten Hochsiedern weniger Wasser enthält. Soll die Entsorgung beispielsweise durch Zugeben zu einer Verbrennung erfolgen, ist für die Entsorgung von Retentatstoffgemisch weniger Energie erforderlich, da weniger Wasser mit verdampft werden muss als wenn die gleiche Menge an Katalysator, Trimethylamin und/oder unerwünschten Hochsiedern durch Zugeben von zweitem Destillationsstoffgemisch zu der Verbrennung erfolgen würde.

Durch die Umwandlung von Dimethylamin in Trimethylamin steigt der Gehalt an Trimethylamin im zweiten Destillationsstoffgemisch zu Lasten des Gehaltes an Dimethylamin durch das Rückführen von zweitem Destillationsstoffgemisch und/oder Retantatstoffgemisch zum Reaktor 1 an. Eine zu große Abnahme an Dimethylamin zugunsten von Trimethylamin verschlechtert die Methacroleinausbeute im Reaktor 1 entsprechend bzw. erfordert eine entsprechende Erhöhung der Reaktionstemperatur, um dem Absinken der Methacroleinausbeute im Reaktor 1 gut zu begegnen. Eine zu hohe Reaktionstemperatur fördert allerdings die Bildung unerwünschter Nebenprodukte. Die Menge an Trimethylamin, welche zum Reaktor 1 zurückgeführt wird, kann insbesondere dadurch verringert werden, dass zweites Destillationsstoffgemisch über den dritten Abschnitt 31 der zweiten Destillationskolonnenabführleitungsanordnung 15 abgeführt wird und/oder Retentatstoffgemisch über den dritten Abschnitt 38 der ersten Membrananlagenabführleitungsanordnung 35 abgeführt wird. Damit verlorengegangener Katalysator kann durch das Zuführen von frischem Katalysator zum Reaktor 1 ausgeglichen werden.

Die Menge an Wasser, die in den Reaktor 1 zurückgeleitet werden kann, ist begrenzt. Wenn die im anfallenden zweiten Destillationsstoffgemisch enthaltene Wassermenge höher ist, als die Wassermenge, die in den Reaktor 1 zurückgeführt werden kann oder sollte, und daher nicht alles anfallende zweite Destillationsstoffgemisch in den Reaktor 1 zurückgeführt werden kann oder sollte, kann nicht aller im anfallenden zweiten Destillationsstoffgemisch enthaltener Katalysator wiederverwendet werden. Die Menge des im anfallenden zweiten Destillationsstoffgemisch enthaltenen Katalysators, der in den Reaktor 1 zurückgeführt werden kann, lässt sich dadurch erhöhen, dass die Menge des in den Reaktor 1 zurückgeführten zweiten Destillationsstoffgemisches reduziert und die Menge des in den Reaktor 1 eingeleiteten Retentatstoffgemisches erhöht wird.

Mit dem aus dem ersten Abschnitt 29 der zweiten Destillationskolonnenabführleitungsanordnung 15 und/oder dem zweiten Abschnitt 37 der Membrananlagenabführleitungsanordnung 35 in den zweiten Abschnitt 30 der zweiten Destillationskolonnenabführleitungsanordnung 15 eingeleiteten Stoffgemisch bzw. Stoffgemischen werden insbesondere nicht umgesetztes Formaldehyd, Wasser und Katalysator in den Reaktor 1 zurückgeführt.

Dem Reaktor 1 kann frischer Katalysator zugeführt werden, indem eine entsprechende Menge an Säure bzw. Säuren und Basen bzw. Basen aus der Säurenquelle 46 und Basenquelle 44 in den Reaktor 1 geleitet wird.

Die Edukte Formaldehyd und Propionaldehyd werden dem Reaktor 1 aus der Formaldehydquelle 30 und der Propionaldehydquelle 42 zugeleitet.

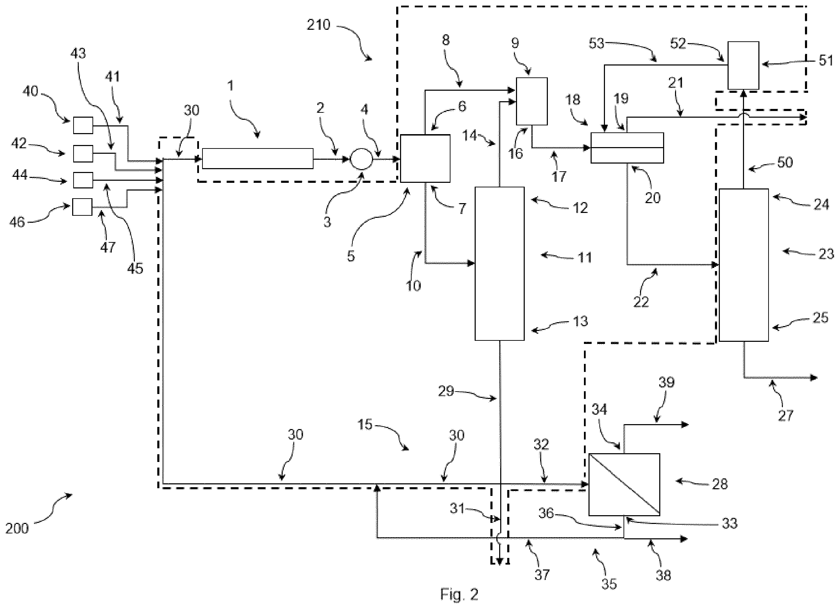

Es wird Bezug auf Figur 2 genommen. Die darin schematisch in einem Fließschema veranschaulichte erfindungsgemäße Herstellanlage 200 der zweiten Ausführungsform der Erfindung unterscheidet sich nur partiell von der Herstellanlage 100 der ersten Ausführungsform der Erfindung. Nachfolgend wird nur auf Unterschiede eingegangen.

Im Unterschied zur Herstellanlage 100 der ersten Ausführungsform der Erfindung hat die Herstellanlage 200 der zweiten Ausführungsform eine Methacroleinaufarbeitungsanlage 210, welche zusätzlich einen zweiten Kondensator 51 aufweist. Dabei ist eine dritte Destillationskolonnenabführleitungsanordnung 50 vorgesehen, welche im Gegensatz zu der dritten Destillationskolonnenabführleitungsanordnung 26 der ersten Ausführungsform der Erfindung den Kopf 24 der zweiten Destillationskolonne 23 nicht fluidisch mit dem ersten Kondensator 9, sondern fluidisch mit dem zweiten Kondensator 51 verbindet, wobei drittes Destillationsstoffgemisches vom Kopf 24 der zweiten Destillationskolonne 23 durch die dritte Destillationskolonnenabführleitungsanordnung 50 der zweiten Ausführungsform der Erfindung in den zweiten Kondensator 51 geleitet werden kann. In dem zweiten Kondensator 51 kann das dritte Destillationsstoffgemisches kondensiert werden. Der zweite Kondensator 51 hat einen Kondensatausgang 52, welcher durch eine zweite Kondensatorabführleitungsanordnung 53 fluidisch mit dem ersten Phasentrenner 18 verbunden ist, wobei Kondensat des zweiten Kondensators 51 durch die zweite Kondensatorabführleitungsanordnung 53 vom zweiten Kondensator 51 in den ersten Phasentrenner 18 geleitet werden kann.

Mit dem zweiten Kondensator 51 kann das dritte Destillationsstoffgemisch unabhängig vom ersten Kondensator 9 kondensiert werden. Da der zweite Kondensator 51 fluidisch mit dem ersten Phasentrenner 18 verbunden ist, kann der erste Phasentrenner 18 auch zum weiteren Aufarbeiten des Kondensates des zweiten Kondensators 51 genutzt werden. D. h. es ist kein zweiter Phasentrenner nötig.

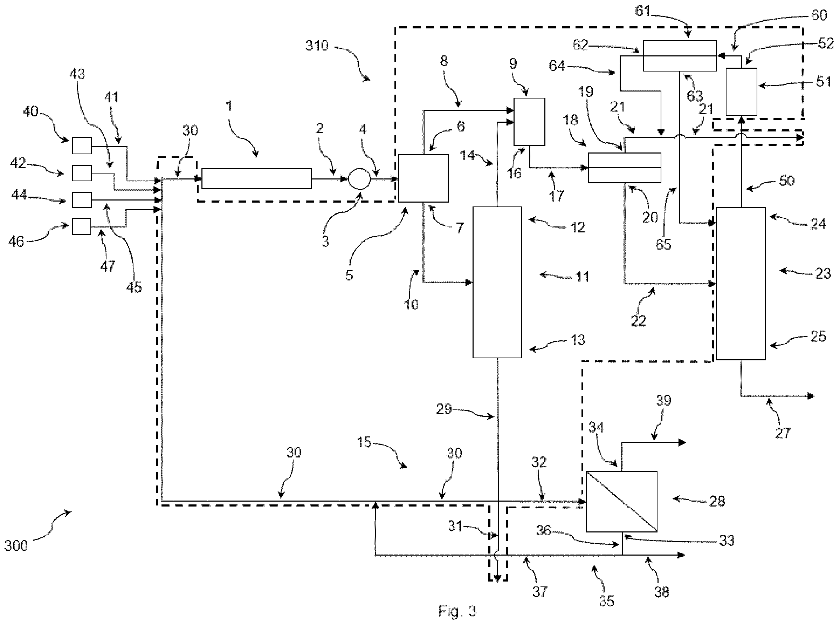

Es wird Bezug auf Figur 3 genommen. Die darin schematisch in einem Fließschema veranschaulichte Herstellanlage 300 der dritten Ausführungsform der Erfindung unterscheidet sich nur partiell von der Herstellanlage 200 der zweiten Ausführungsform der Erfindung. Nachfolgend werden nur Unterschiede erläutert.

Im Unterschied zu der in Figur 2 gezeigten Herstellanlage 200 der zweiten Ausführungsform der Erfindung weist die Methacroleinaufarbeitungsanlage 310 der Herstellanlage 300 der dritten Ausführungsform der Erfindung zusätzlich einen zweiten Phasentrenner 61 auf, mit welchem eine im Kondensat des zweiten Kondensator 51 enthaltene organische Phase (drittes Trennstoffgemisch) von einer im Kondensat des zweiten Kondensators 51 enthaltenen wässrigen Phase (viertes Trennstoffgemisch) getrennt werden kann. Dabei ist eine zweite Kondensatorabführleitungsanordnung 60 vorgesehen, welche den Kondensatausgang 52 des zweiten Kondensators 51 anstatt mit dem ersten Phasentrenner 18 mit dem zweiten Phasentrenner 61 fluidisch verbindet, wodurch Kondensat des zweiten Kondensators 51 vom zweiten Kondensator 51 durch die zweite Kondensatorabführleitungsanordnung 60 in den zweiten Phasentrenner 61 geleitet werden kann. Der zweite Phasentrenner 61 hat einen ersten Ausgang 62 zum Abführen der abgetrennten organischen Phase, also zum Abführen von drittem Trennstoffgemisch, sowie einen zweiten Ausgang 63 zum Abführen der wässrigen Phase, also zum Abführen von viertem Trennstoffgemisch. Der erste Ausgang 62 des zweiten Phasentrenners 61 ist durch eine dritte Phasentrennerabführleitungsanordnung 64 fluidisch mit der ersten Phasentrennerabführleitungsanordnung 21 verbunden, wodurch drittes Trennstoffgemisch durch die dritte Phasentrennerabführleitungsanordnung 64 in die erste Phasentrennerabführleitungsanordnung 21 eingeleitet und zusammen mit erstem Trennstoffgemisch durch den sich stromabseitige an die Einmündungsstelle anschließenden Abschnitt der ersten Phasentrennerabführleitungsanordnung 21 abgeführt werden kann.

Kondensat des zweiten Kondensators 51 wird in den zweiten Phasentrenner 61 geleitet. Dort werden drittes und viertes Trennstoffgemisch getrennt, wobei das dritte Trennstoffgemisch mehr Methacrolein enthält als das vierte Trennstoffgemisch und das vierte Trennstoffgemisch mehr Wasser enthält als das dritte Trennstoffgemisch. Das dritte Trennstoffgemisch hat ebenso wie das erste Trennstoffgemisch einen guten Methacroleingehalt und kann zur Weiterverarbeitung verwendet werden, beispielsweise zum Herstellen von Methylmethacrolein.

In der dritten Ausführungsform der Erfindung wird das dritte Trennstoffgemisch durch die dritte Phasentrennerabführleitungsanordnung 64 und durch den sich stromabseitig an die Einmündungsstelle anschließenden Abschnitt der ersten Phasentrennerabführleitungsanordnung 21 geleitet. D. h. in der dritten Ausführungsform der Erfindung werden erstes und drittes Trennstoffgemisch zusammengeführt.

Der zweite Ausgang 63 des zweiten Phasentrenners 61 ist durch eine vierte Phasentrennerabführleitungsanordnung 65 fluidisch mit der zweiten Destillationskolonne 23 verbunden, wobei viertes Trennstoffgemisch durch die vierte Phasentrennerabführleitungsanordnung 65 vom zweiten Phasentrenner 61 in die zweite Destillationskolonne 23 geleitet werden kann. Durch das Einleiten vierten Trennstoffgemisches in die zweite Destillationskolonne 23 kann im vierten Trennstoffgemisch enthaltenes Methacrolein von im vierten Trennstoffgemisch enthaltenem Wasser getrennt werden.

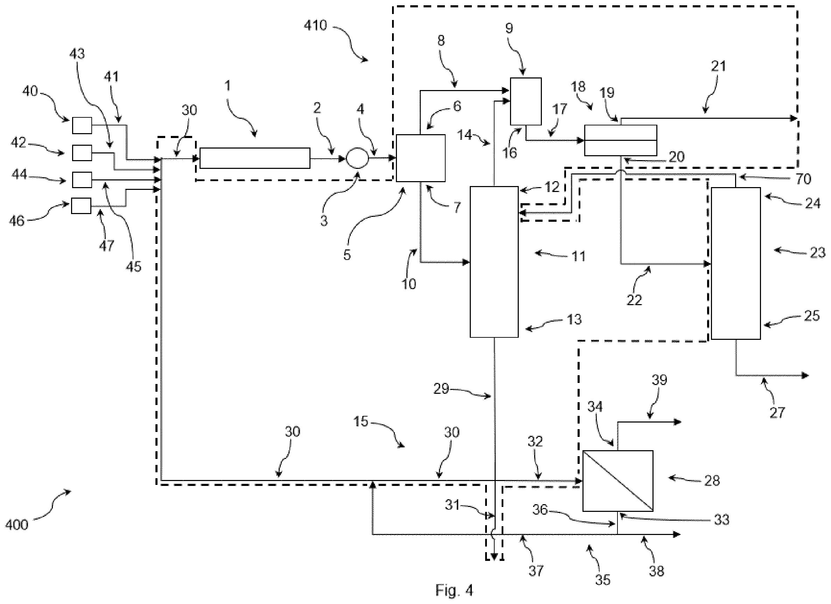

Es wird Bezug auf Figur 4 genommen. Die in Figur 4 schematisch veranschaulichte vierte Ausführungsform der Erfindung unterscheidet sich nur partiell von der in Figur 1 gezeigten ersten Ausführungsform der Erfindung. Nachfolgend werden nur Unterschiede der vierten Ausführungsform der Erfindung gegenüber der ersten Ausführungsform der Erfindung erläutert.

Bei der Herstellanlage 400 der vierten Ausführungsform der Erfindung ist eine dritte Destillationskolonnenabführleitungsanordnung 70 vorgesehen, welche den Kopf 24 der zweiten Destillationskolonne 23 anstatt mit dem ersten Kondensator 9 mit der ersten Destillationskolonne 11 der Methacroleinaufarbeitungsanlage 410 der vierten Ausführungsform der Erfindung fluidisch verbindet, wobei drittes Destillationsstoffgemisch vom Kopf 24 der zweiten Destillationskolonne 23 durch die dritte Destillationskolonnenabführleitungsanordnung 70 in die erste Destillationskolonne 11 geleitet werden kann. Durch das Einleiten von drittem Destillationsstoffgemisch in die erste Destillationskolonne 11 wird das dritte Destillationsstoffgemisch der entsprechenden weiteren Aufarbeitung zugeführt. Zwar enthält das dritte Destillationsstoffgemisch auch Wasser. Jedoch ist der hiermit verbundene Wassereintrag in die erste Destillationskolonne 11 verkraftbar und ist deutlich geringer als wenn zweites Trennstoffgemisch in die erste Destillationskolonne 11 eingeleitet werden würde. Die Vorteile, die dadurch erreicht werden, dass das zweite Trennstoffgemisch nicht in die erste Destillationskolonne 11, sondern in die zweite Destillationskolonne 23 eingeleitet wird, bleiben in einem guten Maße erhalten.

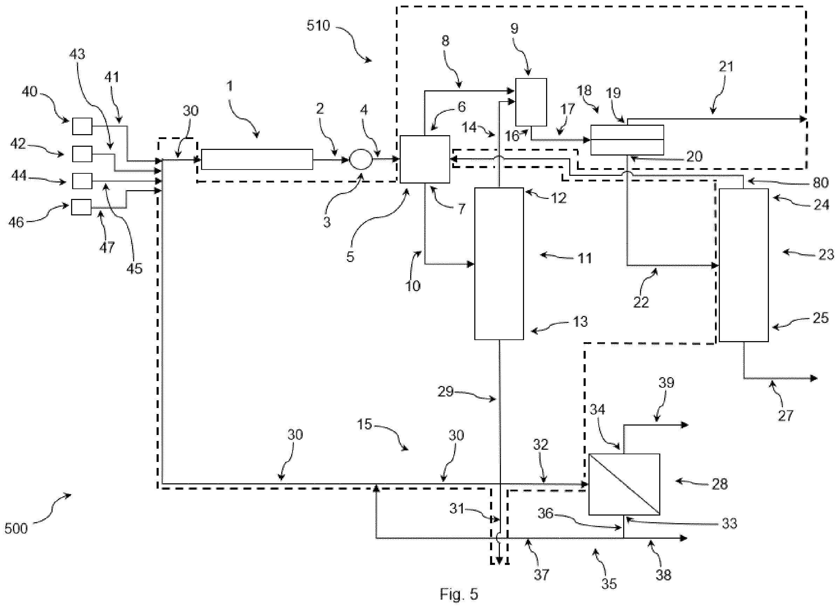

Es wird Bezug auf Figur 5 genommen. Die fünfte Ausführungsform der Erfindung unterscheidet sich nur partiell von der vierten Ausführungsform der Erfindung. Nachfolgend wird nur auf Unterschiede eingegangen.

Bei der Herstellanlage 500 der fünften Ausführungsform der Erfindung ist im Gegensatz zur Herstellanlage 400 der vierten Ausführungsform der Erfindung eine dritte Destillationskolonnenabführleitungsanordnung 80 vorgesehen, welche den Kopf 24 der dritten Destillationskolonne 23 anstatt mit der ersten Destillationskolonne 11 mit dem Entspannungsbehälter 5 verbindet. Durch diese dritte Destillationskolonnenabführleitungsanordung 80 kann drittes Destillationsstoffgemisches vom Kopf 24 der zweiten Destillationskolonne 23 in den Entspannungsbehälter 5 geleitet werden. Durch das Einleiten von drittem Destillationsstoffgemisch in den Entspannungsbehälter 5 wird das dritte Destillationsstoffgemisch der entsprechenden weiteren Aufarbeitung zugeführt. Insbesondere strömt das dritte Destillationsstoffgemisch, da es gasförmig ist, zusammen mit dem ersten Entspannungsstoffgemisch durch die erste Entspannungsbehälterabführleitungsanordnung 8 in den ersten Kondensator 9 und wird dort kondensiert. Als Teil des Kondensates des ersten Kondensators 9 wird das kondensierte dritte Destillationsstoffgemisches über die erste Kondensatorabführleitungsanordnung 17 in den ersten Phasentrenner 18 geleitet und durchläuft das dortige Trennverfahren.

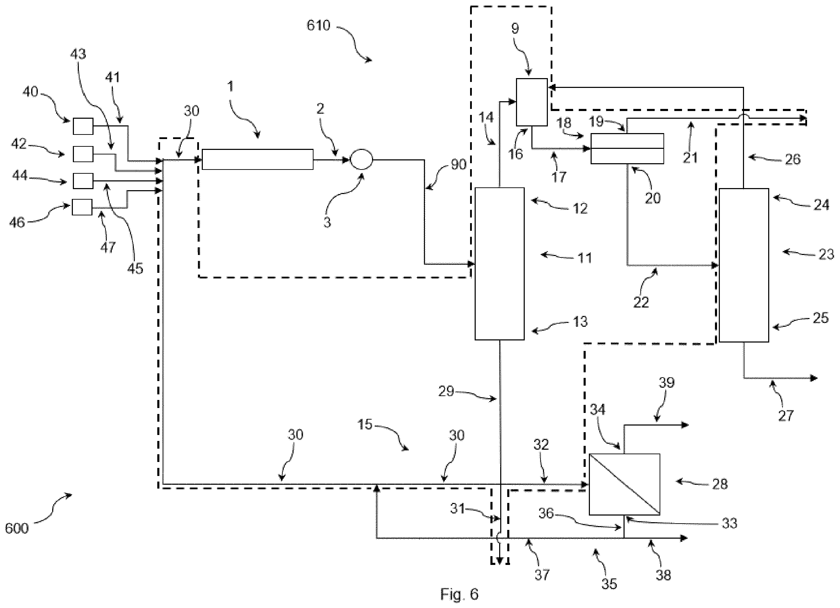

Es wird Bezug auf die Figuren 6 bis 9 genommen. Die darin gezeigten Herstellanlagen 600, 700, 800, 900 der sechsten, siebenten, achten und neunten Ausführungsform weisen Methacroleinaufarbeitungsanlagen 610, 710, 810, 910 auf, welche keinen Entspannungsbehälter 5 haben und folglich auch keine ersten und zweiten Entspannungsbehälterabführleitungsanordnungen 8, 10. In diesen Ausführungsformen ist eine Entspannungsventilabführleitungsanordnung 90 vorgesehen, welche das Entspannungsventil 3 mit der ersten Destillationskolonne 11 fluidisch verbindet. Dadurch kann entspanntes Reaktionsgemisches in die erste Destillationskolonne 11 geleitet werden. Es wird also in die erste Destillationskolonne 11 hinein entspannt. Ansonsten entspricht
- die Herstellanlage 600 der sechsten Ausführungsform der Erfindung der Herstellanlage 100 der ersten Ausführungsform der Erfindung,
- die Herstellanlage 700 der siebenten Ausführungsform der Erfindung der Herstellanlage 200 der zweiten Ausführungsform der Erfindung,
- die Herstellanlage 800 der achten Ausführungsform der Erfindung der Herstellanlage 300 der dritten Ausführungsform der Erfindung und
- die Herstellanlage 900 der neunten Ausführungsform der Erfindung der Herstellanlage 400 der vierten Ausführungsform der Erfindung.

Bei der sechsten bis und neunten Ausführungsform der Erfindung ist die Zusammensetzung des ersten Destillationsstoffgemisches ähnlich der Zusammensetzung des ersten Destillationsstoffgemisches der ersten bis fünften Ausführungsform der Erfindung und die Zusammensetzung des zweiten Destillationsstoffgemisches ähnlich der Zusammensetzung des zweiten Destillationsstoffgemisches der ersten bis fünften Ausführungsform der Erfindung.

Da in der sechsten bis neunten Ausführungsform der Erfindung kein Entspannungsbehälter vorgesehen ist, strömt eine größere Gasmenge durch die ersten Destillationskolonnen dieser Ausführungsformen der Erfindung. Zur Anpassung der ersten Destillationskolonnen hieran können diese beispielsweise nach oben breiter ausgeführt sein und/oder zusätzlich einen Seitenabzug für die Gasphase aufweisen.

Nachfolgend werden weitere Variationsmöglichkeiten beschrieben, die je einzeln oder in beliebigen Kombinationen realisiert werden können.

Es sind Herstellanlagen möglich, welche mehrere oder alle der ersten, zweiten, dritten, vierten und fünften Ausführungsform miteinander kombinieren, sowie Herstellanlagen, welche mehrere oder alle der sechsten, siebenten, achten und neunten Ausführungsform miteinander kombinieren.

Einzelne oder alle Destillationskolonnen können mit einem Reboilerkreislauf aufweisen, durch welchen jeweiliges Sumpfstoffgemisch geleitet und in welchem dieses erwärmt wird.

Einzelne oder alle Destillationskolonnen können einen zur jeweiligen Methacroleinaufarbeitungsanlage separaten Kondensatorkreislauf aufweisen, mit welchem Kopfstoffgemisch kondensiert und in die betreffende Destillationskolonne zurückgeleitet wird, wobei der separate Kondensatorkreislauf einen eigenen Kondensator hat.

Es ist auch möglich, für das Kondensieren des Kopfstoffgemisches der ersten Destillationskolonne den ersten Kondensator 9 mitzubenutzen, d. h. in diesem Fall wäre kein separater Kondensator erforderlich. Zudem ist es möglich, für das Kondensieren des Kopfstoffgemisches der zweiten Destillationskolonnen der zweiten, dritten, siebenten und/oder achten Ausführungsform der Erfindung den jeweiligen zweiten Kondensator 51 mitzubenutzen, d. h. auch in diesem Fall wäre kein separater Kondensator erforderlich.

Einzelne oder alle Destillationskolonnen können jeweils eine Tröpfchenrückhalteeinrichtung aufweisen, mit welcher Tropfen und Tröpfchen daran gehindert werden können, die betreffende Destillationskolonne über deren Kopf zu verlassen. Die Tröpfchenrückhalteeinrichtung kann beispielsweise als feinmaschiges Sieb ausgebildet sein, als sogenannter Demister oder als eine Flüssigkeitsabgabeeinrichtung, mit welcher Flüssigkeit in Richtung des Sumpfes der betreffenden Destillationskolonne im Innernen der Destillationskolonne abgegeben wird, wobei diese Flüssigkeit aufsteigende Tropfen und Tröpfchen einfängt, wodurch die Flüssigkeit der Tropfen und Tröpfchen dann in Richtung Sumpf bewegt wird. Durch die Tröpfchenrückhalteeinrichtung wird die Trenneffizienz der betreffenden Destillationskolonne erhöht.

Die zweite Destillationskolonne 23 kann eine Packungskolonne oder eine Bodenkolonne oder eine Mischform hiervon sein.

Einzelne oder alle Destillationskolonnen können Füllkörper und/oder strukturierte Packungen enthalten.

Einer oder mehrere der Kondensatoren 9, 51 können eine Entlüftung aufweisen, über welche Abgas einer oder mehrerer Reaktionen abgeführt werden kann.

Einer oder mehrere der Kondensatoren 9, 51 können mehrstufig ausgebildet sein, beispielsweise zweistufig. Bei Zweistufigkeit könnte mit der ersten Kondensatorstufe beispielsweise eine Abkühlung auf etwa 30 bis 40 °C erfolgen und mit der zweiten Kondensatorstufe beispielsweise eine Abkühlung auf etwa 3 bis 10 °C, vorzugsweise auf 4 °C. Durch die Mehrstufigkeit wird Kühlsole eingespart.

Wenn mehrstufige Kondensatoren eingesetzt werden, kann ein Teil des Kondensates in die erste Kondensationsstufe rückgeführt und mit einem gegen Polymerisation wirkenden Stabilisator versetzt werden.

Einer oder mehrere der Kondensatoren 9, 51 können eine Gasphasenabführeinrichtung haben, mit welcher eine in dem betreffenden Kondensator vorliegende Gasphase aus diesem Kondensator abgeführt werden kann.

Zweites Trennstoffgemisch kann mit einem Mittel versetzt werden, welches Polymerisation entgegenwirkt, beispielsweise der Polymerisation von Methacrolein und/oder von Dimethylamin. Dieses modifizierte zweite Trennstoffgemisch kann in einen oder mehrere Kondensatoren eingeleitet werden, um bereits dort Polymerisation entgegenzuwirken.

Die in den Reaktor 1 eingeleiteten Stoffe können beispielsweise auf eine Temperatur von etwa 130 °C vorgewärmt werden.

Der Reaktor 1 kann als Rohrreaktor ausgebildet sein.

Die in den Reaktor 1 eingeleitete Base bzw. Basen aus der Basenquelle 44, Säure bzw. Säuren aus der Säurenquelle 46 und/oder der Rückführstoffstrom, der vom ersten Abschnitt 29 der zweiten Destillationskolonenabführleitungsanordnung 15 und/oder dem zweiten Abschnitt 37 der ersten Membrananlagenabführleitungsanordnung 35 kommend durch den zweiten Abschnitt 30 der zweiten Destillationskolonnenabführleitungsanordnung 15 in den Reaktor 1 eingeleitet wird, kann bzw. können stärker vorgewärmt werden als das frische Formaldehyd und das frische Propionaldehyd, welche aus der Formaldehydquelle 40 und der Propionaldehydquelle 42 in den Reaktor 1 eingeleitet werden. Dabei kann die Säure bzw. die Säuren, die Base bzw. die Basen und/oder der genannte Rückführstoffstrom so stark erhitzt werden, dass erst nach ihrer Vermischung mit dem frischen Formaldehyd und dem frischen Propionaldehyd eine angestrebte Vormischtemperatur erreicht wird. So bleiben das frische Formaldehyd und das frische Propionaldehyd länger bei einer Temperatur, die niedriger ist als die gewünschte Vormischtemperatur.

Auch der genannte Rückführstoff kann vorgewärmt werden.

Dem as dem Sumpf 13 der ersten Destillationskolonne 11 abgeführten zweiten Destillationsstoffgemisch kann Wärme entzogen werden, welche wenigstens teilweise zum Vorwärmen wenigstens eines Teiles der in den Reaktor 1 eingeleiteten Stoffe genutzt werden kann.

Einzelne oder alle Entspannungsbehälter 5 - soweit vorhanden - können eine Tröpfchenrückhalteeinrichtung aufweisen, mit welcher Tropfen und Tröpfchen zweiten Entspannungsstoffgemisches daran gehindert werden können, den Entspannungsbehälter 5 zusammen mit erstem Entspannungsstoffgemisch über dessen ersten Ausgang 6 zu verlassen. Die Tröpfchenrückhalteeinrichtung kann beispielsweise als feinmaschiges Sieb ausgebildet sein. Durch die Tröpfchenrückhalteeinrichtung wird die Trenneffizienz des Entspannungsbehälters 5 erhöht. Dementsprechend wird auch die Menge an Katalysator, die den Entspannungsbehälter 5 über seinen ersten Ausgang 6 verlässt, verringert.

Die Tröpfchenrückhalteeinrichtung des Entspannungsbehälters 5 kann als sogenannter Demister ausgebildet sein.

Der Entspannungsbehälter 5 kann eine Sprüheinrichtung aufweisen, mit welcher die Tröpfchenrückhalteeinrichtung von unten mit einer Flüssigkeit besprüht werden kann, um die Wirkung der Tröpfchenrückhalteeinrichtung zu erhöhen. Bei der Flüssigkeit kann es sich beispielsweise um zweites Entspannungsstoffgemisch handeln oder um eine Flüssigkeit, welche ein Mittel enthält, welches der Polymerisation von Methacrolein und/oder Dimethylamin entgegenwirkt.

Bei Bedarf kann beispielsweise in den ersten Kondensator 9 und/oder den zweiten Abschnitt 30 der zweiten Destillationskolonnenabführleitungsanordnung 15 Stabilisator zugegeben werden.

In der ersten bis fünften Ausführungsform der Erfindung ist das Entspannungsventil 3, wie in den Figuren 1 bis 5 gezeigt, außerhalb des Entspannungsbehälters 5 angeordnet. Es ist jedoch auch möglich, das Entspannungsventil 3 im Inneren des Entspannungsbehälters 5 anzuordnen oder es in dessen Wandung zu integrieren. In solchen Fällen sind auch Ausgestaltungen möglich, bei welchen auf eine Entspannungsventilabführleitungsanordnung verzichtet werden kann.

In der sechsten bis neunten Ausführungsform der Erfindung ist das Entspannungsventil 3, wie in den Figuren 6 bis 9 gezeigt, vor der ersten Destillationskolonne 11 angeordnet. Es ist jedoch auch möglich, es im Inneren der ersten Destillationskolonne 11 oder in die Wandung der ersten Destillationskolonne 11 integriert vorzusehen. Dabei sind auch Varianten möglich, bei welchen auf eine Entspannungsventilabführleitungsanordnung verzichtet werden kann.

In der ersten bis fünften Ausführungsform der Erfindung führen die erste Entspannungsbehälterabführleitungsanordnung 8 und die erste Destillationskolonnenabführleitungsanordnung 14 separat in den ersten Kondensator 9. Es ist jedoch auch möglich, dass sich die erste Entspannungsbehälterabführleitungsanordnung 8 und die erste Destillationskolonnenabführleitungsanordnung 14 vereinigen und vereinigt an einen gemeinsamen Eingang des ersten Kondensators 9 angeschlossen sind, d. h. fluidisch mit diesem gemeinsamen Eingang verbunden sein.

In der ersten und sechsten Ausführungsform der Erfindung führt die dritte Destillationskolonnenabführleitungsanordnung 26 separat in den ersten Kondensator. Es ist jedoch auch möglich, dass sich die dritte Destillationskolonnenabführleitungsanordnung 26 und die erste Destillationskolonnenabführleitung 24 vereinigen und vereinigt an einen gemeinsamen Eingang des ersten Kondensators 9 angeschlossen sind, d. h. fluidisch mit diesem gemeinsamen Eingang verbunden sind.

In der ersten bis neunten Ausführungsform der Erfindung ist eine zweite Destillationskolonnenabführleitungsanordnung 15 vorgesehen. Es können jedoch auch zwei separate zweite Destillationskolonnenabführleitungsanordnungen vorgesehen sein, wobei eine den ersten und zweiten und optional dritten Abschnitt 29, 30, 31 der zweiten Destillationskolonnenabführleitungsanordnungen 15 der ersten bis neunten Ausführungsformen der Erfindung aufweist und folglich den Sumpf 13 der ersten Destillationskolonne 11 insbesondere mit dem Reaktor 1 fluidisch verbindet, und die andere den Sumpf 13 der ersten Destillationskolonne 11
mit der Membrananlage 28 fluidisch verbindet.

In der zweiten, dritten, siebenten und achten Ausführungsform der Erfindung verbindet die dritte Destillationskolonnenabführleitungsanordnung 50 den Kopf 24 der zweiten Destillationskolonne 23 nur mit dem zweiten Kondensator 51. Es ist auch möglich, eine dritte Destillationskolonnenabführleitungsanordnung vorzusehen, welche den Kopf 24 der zweiten Destillationskolonne 23 sowohl mit dem ersten Kondensator 9 als auch mit dem den zweiten Kondensator 51 fluidisch verbindet.

In der dritten und achten Ausführungsform der Erfindung werden erstes und drittes Trennstoffgemisch zusammengeführt. Es ist jedoch auch möglich, die dritte Phasentrennerabführleitungsanordnung so vorzusehen, dass sie nicht mit der ersten Phasentrennerabführleitungsanordnung 21 fluidisch verbunden ist, sondern separat zu dieser an einen Tank oder eine weiterverarbeitende Anlage fluidisch angeschlossen ist.

Die Membrananlage 28 ist stets optional und kann weggelassen werden.

Die Membrananlage 28 kann ein- oder mehrstufig ausgebildet sein.

Vorzugsweise ist die Membrananlage eine dreistufige Umkehrosmosemembrananlage, deren erste Stufe in einem Druckbereich von 80 bis 120 bar (absolut) betrieben wird, deren zweite Stufe in einem Druckbereich von 20 bis 80 bar (absolut) betrieben wird und deren dritte Stufe in einem Druckbereich von 20 bis 40 bar (absolut) betrieben wird. Wenn als Base Dimethylamin eingesetzt wird und als Säure Essigsäure, können mit einer so betriebenen Umkehrosmosemembrananlage mehr als 99 % des im zweiten Destillationsstoffgemisch enthaltenen Dimethyamins, ca. 80 % von im zweiten Destillationsstoffgemisch enthaltener Essigsäure und ca. 70 bis 80 % des im zweiten Destillationsstoffgemisch enthaltenen Formaldehyds zurückgehalten werden.

Die Membrananlage 28 kann mit einer Kühlvorrichtung versehen sein, mit welcher insbesondere die Arbeitstemperatur der Membrananlage reguliert werden kann.

In der ersten bis neunten Ausführungsform der Erfindung sind die Formaldehydzuleitungsanordnung 41, die Propionaldehydzuleitungsanordnung 43, die Basenzuleitungsanordnung 45 und die Säurenzuleitungsanordnung 47 fluidisch mit dem zweiten Abschnitt 30 der zweiten Destillationskolonnenabführleitungsanordnung 15 verbunden. Alternativ ist es möglich, die Formaldehydzuleitungsanordnung 41, die Propionaldehydzuleitungsanordnung 43, die Basenzuleitungsanordnung 45 und/oder die Säurenzuleitungsanordnung 47 fluidisch direkt mit dem Reaktor 1 zu verbinden, wodurch Formaldehyd, Propionaldehyd, Base(n) und/oder Säure(n) direkt in den Reaktor 1 eingeleitet werden kann bzw. können.

In der ersten bis neunten Ausführungsform der Erfindung werden frisches Formaldehyd, frisches Propionaldehyd, frische Säure bzw. Säuren und frische Base bzw. Basen aus separaten Quellen zugeführt. Es ist auch möglich,
- frisches Formaldehyd und frisches Propionaldehyd vorzumischen oder ein Gemisch mit Formaldehyd und Propionaldehyd aus einer entsprechenden Quelle zuzuführen und/oder
- frische Säure bzw. Säuren und frische Base bzw. Basen vorzumischen oder ein Gemisch mit Säure bzw. Säuren und Base bzw. Basen aus einer entsprechenden Quelle zuzuführen.

Wenn keine Vormischung von Säure bzw. Säuren und Base bzw. Basen erfolgt, kann zuerst Säure bzw. Säuren in den zweiten Abschnitt 30 der zweiten Destillationskolonnenabführleitungsanordnung 15 geleitet werden, bevor Base bzw. Basen in den zweiten Abschnitt 30 der zweiten Destillationskolonnenabführleitungsanordnung 15 geleitet werden. Mit dieser Reihenfolge ist es leichter möglich, einem lokalen Basenüberschuss entgegenzuwirken.

### Erfindunqsqemäßes Versuchsbeispiel

Mit dem Simulationsprogramm *Aspen Plus V8.8* der Firma Aspen Technologies, Inc. wurde der erfindungsgemäße Betrieb einer erfindungsgemäßen Herstellanlage simuliert, und zwar abgesehen von den nachfolgend aufgeführten Abweichungen gemäß der ersten Ausführungsform der Erfindung, wobei trotz dieser Abwandlungen im Folgenden die Bezugszeichen der ersten Ausführungsform der Erfindung verwendet werden:
- Die Herstellanlage hat keine Membrananlage.
- Die Formaldehydzuleitungsanordnung und die Propionaldehydzuleitungsanordnung vereinigen sich zu einer Formaldehyd-Propionaldehyd-Zuleitungsanordnung, welche in den zweiten Abschnitt 30 der zweiten Destillationskolonnenabführleitungsanordnung 15 mündet.
- Der erste Kondensator 9 hat eine Gasphasenabführeinrichtung, welche mit einer Verbrennungsanlage zum Verbrennen der Gasphase fluidisch verbunden ist.
- Der erste Kondensator 9 hat eine vor seinem ersten Ausgang 6 angeordnete Tröpfchenrückhalteeinrichtung, welche als sogenannter Demister ausgebildet ist.
- Der erste Kondensator 9 hat einen Sammeleinlass, welcher die Zuströme von erstem Entspannungsstoffgemisch, erstem Destillationsstoffgemisch und drittem Destillationsstoffgemisch zusammenführt.
- Der erste Kondensator 9 ist zweistufig ausgebildet.

Vom Sumpf 13 der ersten Destillationskolonne 11 her strömt Rückführstoffstrom durch den zweiten Abschnitt 30 der zweiten Destillationskolonnenabführleitungsanordnung 15 in Richtung zu dem Reaktor 1. Von der Säurenquelle 44 wird wässrige Essigsäurelösung über die Säurenzuleitungsanordnung 45 in den zweiten Abschnitt 30 der zweiten Destillationskolonnenabführleitungsanordnung 15 eingeleitet. Von der Basenquelle 46 wird wässrige Dimethylaminlösung über die Basenzuleitungsanordnung 47 in den zweiten Abschnitt 30 der zweiten Destillationskolonnenabführleitungsanordnung 15 eingeleitet. Das so entstandene Stoffgemisch wird auf 130 °C vorgewärmt.

Von der Formaldehydquelle 40 kommend wird wässrige Formaldehydlösung über die Formaldhydzuleitungsanordnung 41 in die Formaldehyd-Propionaldehyd-Zuleitungsanordnung eingeleitet und von der Propionaldehydquelle 42 wird wässrige Propionaldehydlösung wird über die Propionaldhydzuleitungsanordnung 43 in die Formaldehyd-Propionaldehyd-Zuleitungsanordnung eingeleitet. Das so entstehende Gemisch aus Formaldehyd- und Propionaldehydlösung wird auf 130 °C vorgewärmt und in den zweiten Abschnitt 30 der zweiten Destillationskolonnenabführleitungsanordnung 15 eingeleitet. Das nun im zweiten Abschnitt 30 der zweiten Destillationskolonnenabführleitungsanordnung 15 vorliegende Stoffgemisch wird in den Reaktor 1 eingeleitet, welcher in diesem erfindungsgemäßen Versuchsbeispiel als Rohrreaktor ausgebildet ist.

Die Verweilzeit im Reaktor 1 beträgt 9,5 s und der Druck im Reaktor beträgt 35 bar (absolut). Es wird nahezu Vollumsatz erreicht. Das den Reaktor verlassende Reaktionsgemisch hat eine Temperatur von 165 °C. In dem Entspannungsbehälter 5 wird das Reaktionsgemisch auf 0,85 bar (absolut) entspannt.

Die erste Stufe des ersten Kondensators 9 wird mit Kühlwasser gekühlt und seine zweite Stufe mit Kühlsole, die eine Temperatur von 4 °C hat.

Die im ersten Kondensator vorliegende Gasphase wird über die Gasphasenabführeinrichtung einer Verbrennung zugeleitet.

In der nachfolgenden Tabelle 1 sind der Massenstrom, der Druck, die Temperatur und die Zusammensetzung einiger Ströme des erfindungsgemäßen Versuchsbeispiels angegeben. In Tabelle 1
- bedeutet "Strom A": Dimethylaminlösungsstrom durch die Basenzuleitungsanordnung 47,
- bedeutet "Strom B": Essigsäurelösungsstrom durch die Säurenzuleitungsanordnung 45,
- bedeutet "Strom C": Propionaldehydlösungsstrom durch die Propionaldehydzuleitungsanordnung 43,
- bedeutet "Strom D": Formaldehydlösungsstrom durch die Formaldehydzuleitungsanordnung 41,
- bedeutet "Strom E": Strom, der in den Reaktor 1 eintritt,
- bedeutet "Strom F": Reaktionsgemischstrom durch die Reaktorabführleitungsanordnung 2,
- bedeutet "Strom G": Strom ersten Entspannungsstoffgemisches durch die erste Entspannungsbehälterabführleitungsanordnung 8,
- bedeutet "Strom H": Strom zweiten Entspannungsstoffgemisches durch die zweite Entspannungsbehälterabführleitungsanordnung 10,
- bedeutet "Strom I": Strom ersten Destillationsstoffgemisches durch die erste Destillationskolonnenabführleitungsanordnung 14,
- bedeutet "Strom J": Strom zweiten Destillationsstoffgemisches durch den ersten Abschnitt 29 der zweiten Destillationskolonnenabführleitungsanordnung 15,
- bedeutet "Strom K": Strom zweiten Destillationsstoffgemisches, welcher vom ersten Abschnitt 29 der zweiten Destillationskolonnenabführleitungsanordnung 15 in den zweiten Abschnitt 30 der zweiten Destillationskolonnenabführleitungsanordnung 15 strömt,
- bedeutet "Strom L": Strom zweiten Destillationsstoffgemisches, welcher vom ersten Abschnitt 29 der zweiten Destillationskolonnenabführleitungsanordnung 15 in den dritten Abschnitt 31 der zweiten Destillationskolonnenabführleitungsanordnung 15 strömt und einer Entsorgung zugeführt wird,
- bedeutet "Strom M": Strom durch den Sammeleinlass des ersten Kondensators 9,
- bedeutet "Strom N": Strom ersten Trennstoffgemisches durch die erste Phasentrennerabführleitungsanordnung 21,
- bedeutet "Strom O": Strom zweiten Trennstoffgemisches durch die zweite Phasentrennerabführleitungsanordnung 22,
- bedeutet "Strom P": Strom dritten Destillationsstoffgemisches durch die dritte Destillationskolonnenabführleitungsanordnung 26,
- bedeutet "Strom Q": Strom vierten Destillationsstoffgemisches durch die vierte Destillationskolonnenabführleitungsanordnung 27,
- steht die Angabe "%" für Gewichts-% bezogen auf die Gesamtmasse des betreffenden Stromes und
- steht die Anzahl der Kommastellen für die Genauigkeit der Zahlenwerte.

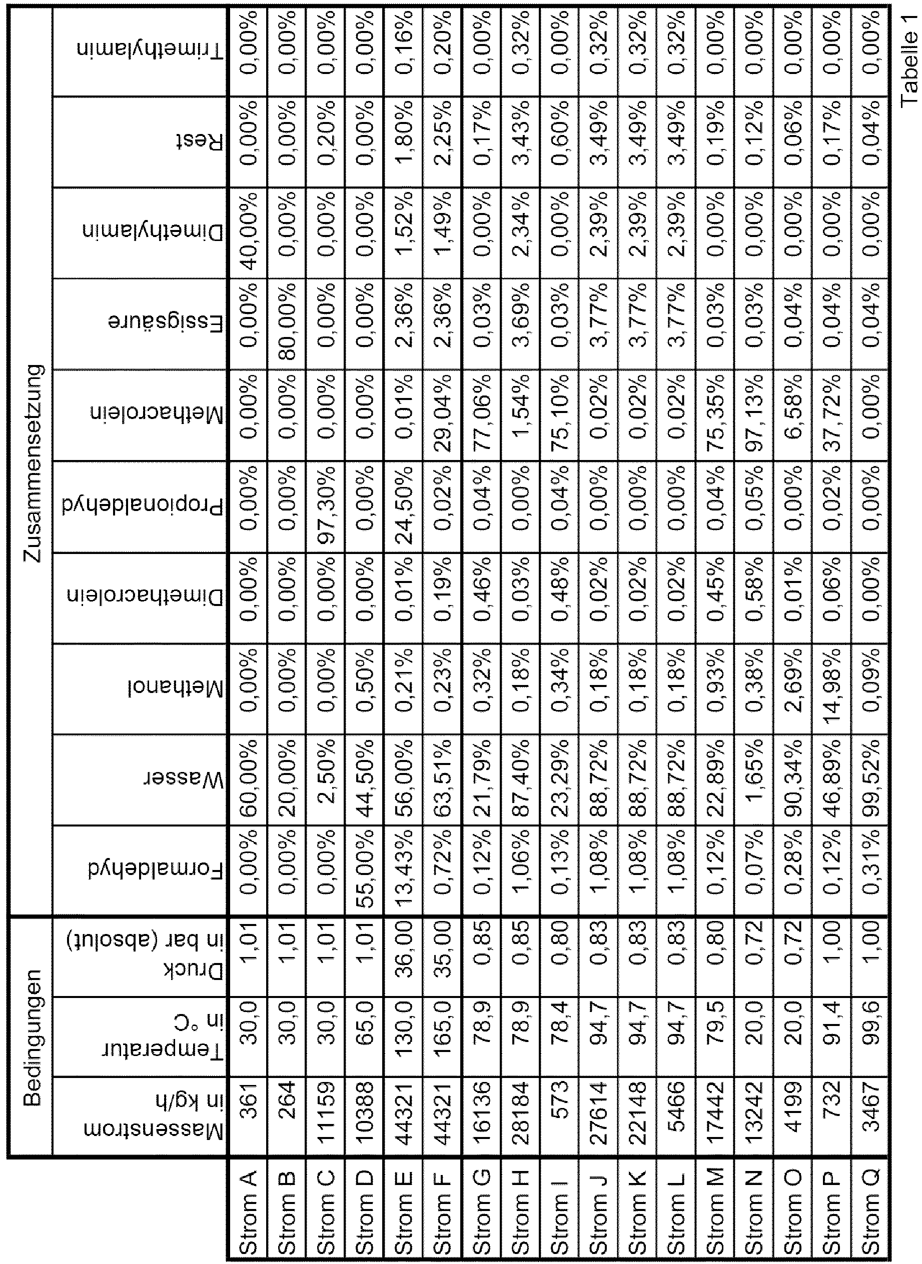

### Nicht-erfindungsgemäßes Vergleichsversuchsbeispiel

Mit dem Simulationsprogramm *Aspen Plus V8.8* der Firma Aspen Technologies, Inc. wurde als nicht-erfindungsgemäßes Vergleichsversuchsbeispiel auch der Betrieb einer nicht-erfindungsgemäßen Herstellanlage simuliert. Im Gegensatz zu dem erfindungsgemäßen Versuchsbeispiel weist die Herstellanlage des nicht-erfindungsgemäßen Vergleichsversuchsbeispiels keine zweite Destillationskolonne auf und die zweite Phasentrennerabführleitungsanordnung ist mit der ersten Destillationskolonne fluidisch verbunden. Das Herstellverfahren des nicht-erfindungsgemäßen Vergleichsversuchsbeispiels weicht dementsprechend vom Herstellverfahren des erfindungsgemäßen Versuchsbeispiels ab. Trotz dieser Abwandlungen werden im Folgenden die Bezugszeichen der ersten Ausführungsform der Erfindung verwendet.

In der nachfolgenden Tabelle 2 sind der Massenstrom, der Druck, die Temperatur und die Zusammensetzung einiger Ströme des nicht-erfindungsgemäßen Vergleichsversuchsbeispiels angegeben. In Tabelle 2
- bedeutet "Strom A": Dimethylaminlösungsstrom durch die Basenzuleitungsanordnung 47,
- bedeutet "Strom B": Essigsäurelösungsstrom durch die Säurenzuleitungsanordnung 45,
- bedeutet "Strom C": Propionaldehydlösungsstrom durch die Propionaldehydzuleitungsanordnung 43,
- bedeutet "Strom D": Formaldehydlösungsstrom durch die Formaldehydzuleitungsanordnung 41,
- bedeutet "Strom E": Strom, der in den Reaktor 1 eintritt,
- bedeutet "Strom F": Reaktionsgemischstrom durch die Reaktorabführleitungsanordnung 2,
- bedeutet "Strom G": Strom ersten Entspannungsstoffgemisches durch die erste Entspannungsbehälterabführleitungsanordnung 8,
- bedeutet "Strom H": Strom zweiten Entspannungsstoffgemisches durch die zweite Entspannungsbehälterabführleitungsanordnung 10,
- bedeutet "Strom I": Strom ersten Destillationsstoffgemisches durch die erste Destillationskolonnenabführleitungsanordnung 14,
- bedeutet "Strom J": Strom zweiten Destillationsstoffgemisches durch den ersten Abschnitt 29 der zweiten Destillationskolonnenabführleitungsanordnung 15,
- bedeutet "Strom K": Strom zweiten Destillationsstoffgemisches, welcher vom ersten Abschnitt 29 der zweiten Destillationskolonnenabführleitungsanordnung 15 in den zweiten Abschnitt 30 der zweiten Destillationskolonnenabführleitungsanordnung 15 strömt,
- bedeutet "Strom L": Strom zweiten Destillationsstoffgemisches, welcher vom ersten Abschnitt 29 der zweiten Destillationskolonnenabführleitungsanordnung 15 in den dritten Abschnitt 31 der zweiten Destillationskolonnenabführleitungsanordnung 15 strömt und einer Entsorgung zugeführt wird,
- bedeutet "Strom M": Strom durch den Sammeleinlass des ersten Kondensators 9,
- bedeutet "Strom N": Strom ersten Trennstoffgemisches durch die erste Phasentrennerabführleitungsanordnung 21,
- bedeutet "Strom O": Strom zweiten Trennstoffgemisches durch die zweite Phasentrennerabführleitungsanordnung 22,
- steht die Angabe "%" für Gewichts-% bezogen auf die Gesamtmasse des betreffenden Stromes und
- steht die Anzahl der Kommastellen für die Genauigkeit der Zahlenwerte.

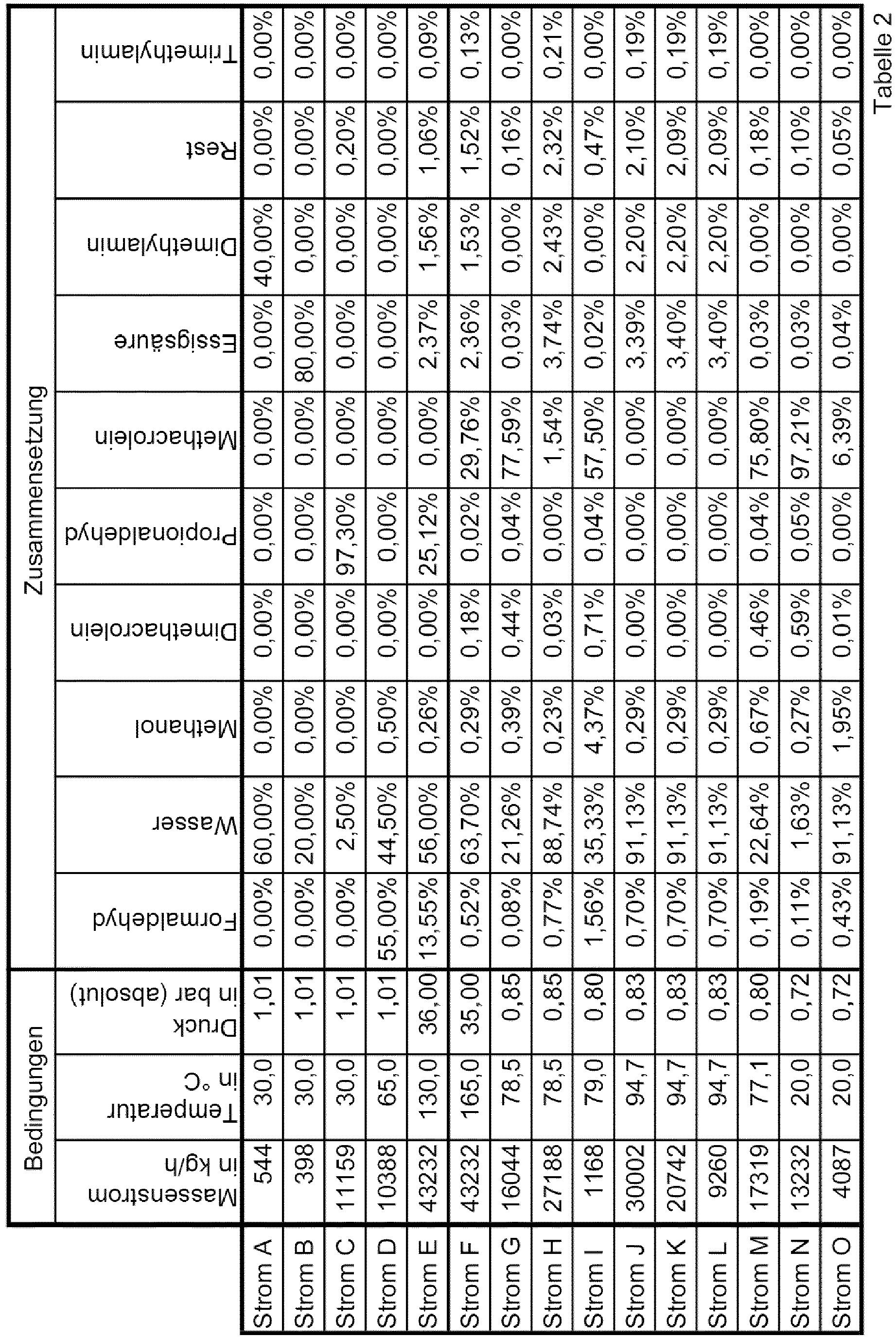

### Vergleich von erfindungsgemäßem Versuchsbeispiel und nicht-erfindungsgemäßem Vergleichsversuchsbeispiel

Vergleicht man das erfindungsgemäße Versuchsbeispiel mit dem nicht-erfindungsgemäßen Vergleichsversuchsbeispiel, wird deutlich, dass der Strom zweiten Destillationsstoffgemisches durch den ersten Abschnitt 29 der zweiten Destillationskolonnenabführleitungsanordnung 15 (Strom J) im erfindungsgemäßen Versuchsbeispiel einen geringeren Massenstrom hat, welcher insbesondere auf eine geringere absolute Wassermenge zurückgeht, und die Konzentration an Dimethylamin und Essigsäure höher ist. Im erfindungsgemäßen Versuchsbeispiel kann also mehr Katalysator pro absoluter Menge Wasser zum Reaktor zurückgeführt werden. Soll zweites Destillationsstoffgemisch entsorgt werden, indem es einer Verbrennung zugeführt wird, ist im erfindungsgemäßen Versuchsbeispiel der Energieaufwand für pro absoluter Menge an Dimethylamin bzw. absoluter Menge an Essigsäure geringer, da pro absoluter Menge an Dimethylamin bzw. absoluter Menge an Essigsäure eine geringere absolute Menge an Wasser verdampft werden muss.

Vergleicht man den Dimethylaminlösungsstrom durch die jeweilige Basenzuleitungsanordnung 47 (Strom A) und den Essigsäurelösungsstrom durch die jeweilige Säurenzuleitungsanordnung 45 (Strom B), wird deutlich, dass im erfindungsgemäßen Versuchsbeispiel deutlich weniger Dimethylamin und deutlich weniger Essigsäure pro an in dem Strom ersten Trennstoffgemisches durch die erste Phasentrennerabführleitungsanordnung 21 (Strom N) enthaltener absoluter Menge Methacrolein eingesetzt wurde.

## Patentansprüche

1. Verfahren zur Herstellung von Methacrolein aus Formaldehyd und Propionaldehyd in Gegenwart von Wasser und in Gegenwart eines homogenen Katalysators, welcher wenigstens auf einer Säure und einer Base basiert, wobei das Verfahren folgende Schritte enthält:
- Schritt S1: Einleiten von Formaldehyd, Propionaldehyd, Wasser und homogenem Katalysator in einen Reaktor (1),
- Schritt S2: in flüssiger Phase Herstellen eines flüssigen Reaktionsgemisches in dem Reaktor (1), wobei das Reaktionsgemisch neben Methacrolein auch Begleitkomponenten enthält, beispielsweise Wasser, Katalysator, nicht umgesetztes Formaldehyd und nicht umgesetztes Propionaldehyd,
- Schritt S3: Einleiten von Reaktionsgemisch in eine Methacroleinaufarbeitungsanlage (110, 210, 310, 410, 510, 610, 710, 810, 910) und wenigstens anteiliges Abtrennen von in dem Reaktionsgemisch enthaltenen Begleitkomponenten in der Methacroleinaufarbeitungsanlage (110, 210, 310, 410, 510, 610, 710, 810, 910), wobei das wenigstens anteilige Abtrennen folgende Schritte enthält:
• Schritt S3.i: Einleiten wenigstens einer Fraktion des Reaktionsgemisches in eine zur Methacroleinaufarbeitungsanlage (110, 210, 310, 410, 510, 610, 710, 810, 910) gehörende erste Destillationskolonne (11) und Trennen dieses eingeleiteten Stoffgemisches wenigstens in ein erstes Destillationsstoffgemisch und ein zweites Destillationsstoffgemisch, wobei das erste Destillationsstoffgemisch am Kopf (12) der ersten Destillationskolonne (11) als Gasphase vorliegt und Methacrolein enthält und das zweite Destillationsstoffgemisch im Sumpf (13) der ersten Destillationskolonne (11) als flüssige Phase vorliegt und Wasser sowie Katalysator enthält,
• Schritt S3.ii: Einführen von vom Kopf (12) der ersten Destillationskolonne (11) abgeführtem ersten Destillationsstoffgemisch in einen zur Methacroleinaufarbeitungsanlage (110, 210, 310, 410, 510, 610, 710, 810, 910) gehörenden ersten Kondensator (9) und Kondensieren des abgeführten ersten Destillationsstoffgemisches in dem ersten Kondensator (9),
• Schritt S3.iii: Abführen von Kondensat aus dem ersten Kondensator (9), Einleiten dieses Kondensates in einen zur Methacroleinaufarbeitungsanlage (110, 210, 310, 410, 510, 610, 710, 810, 910) gehörenden ersten Phasentrenner (18) und Trennen des Kondensates in ein erstes Trennstoffgemisch und ein zweites Trennstoffgemisch in dem ersten Phasentrenner (18), wobei das erste Trennstoffgemisch als organische Phase vorliegt, welche Methacrolein enthält, und das zweite Trennstoffgemisch als wässrige Phase, und
• Schritt S3.iv: Abführen von zweitem Destillationsstoffgemisch aus dem Sumpf (13) der ersten Destillationskolonne (11),
- Schritt S4: Einleiten wenigstens eines Teiles des in Schritt (3.iv) abgeführten zweiten Destillationsstoffgemisches in den Reaktor (1),
- Schritt S5: Einleiten von zweitem Trennstoffgemisch in eine zweite Destillationskolonne (23) und Trennen des eingeleiteten zweiten Trennstoffgemisches wenigstens in ein drittes Destillationsstoffgemisch und ein viertes Destillationsstoffgemisch, wobei das dritte Destillationsstoffgemisch am Kopf (24) der zweiten Destillationskolonne als Gasphase vorliegt und Methacrolein enthält und das vierte Destillationsstoffgemisch im Sumpf (25) der zweiten Destillationskolonne (23) als flüssige Phase vorliegt und Wasser enthält,
- Schritt S6: Abführen von drittem Destillationsstoffgemisch vom Kopf (24) der zweiten Destillationskolonne (23) und Einleiten von abgeführtem dritten Destillationsstoffgemisch in die Methacroleinaufarbeitungsanlage (110, 210, 310, 410, 510, 610, 710, 810, 910) und
- Schritt S7: Abführen von viertem Destillationsstoffgemisch aus dem Sumpf (25) der zweiten Destillationskolonne (23),
wobei der Druck im Reaktor (1) höher ist als in der ersten Destillationskolonne (11).

2. Verfahren nach Anspruch 1, **dadurch gekennzeichnet, dass** in Schritt S6 wenigstens ein Teil des dritten Destillationsstoffgemisches in den ersten Kondensator (9) eingeleitet wird.

3. Verfahren nach Anspruch 1 oder 2, **dadurch gekennzeichnet, dass** in Schritt S6 wenigstens ein Teil des dritten Destillationsstoffgemisches in einen zur Methacroleinaufarbeitungsanlage (210, 710) gehörenden zweiten Kondensator (51) eingeleitet und in diesem kondensiert wird und Kondensat aus dem zweiten Kondensator (51) abgeführt und in den ersten Phasentrenner (18) eingeleitet wird.

4. Verfahren nach einem der vorhergehenden Ansprüche, **dadurch gekennzeichnet, dass** in Schritt S6 wenigstens ein Teil des dritten Destillationsstoffgemisches in einen zur Methacroleinaufarbeitungsanlage (310, 810) gehörenden zweiten Kondensator (51) eingeleitet und in diesem kondensiert wird, Kondensat aus dem zweiten Kondensator (51) abgeführt und in einen zur Methacroleinaufarbeitungsanlage (310, 810) gehörenden zweiten Phasentrenner (61) eingeleitet und in dem zweiten Phasentrenner (51) in ein drittes Trennstoffgemisch und ein viertes Trennstoffgemisch getrennt wird, wobei das dritte Trennstoffgemisch als organische Phase vorliegt, welche Methacrolein enthält, und das vierte Trennstoffgemisch als wässrige Phase.

5. Verfahren nach einem der vorhergehenden Ansprüche, **dadurch gekennzeichnet, dass** in Schritt S6 wenigstens ein Teil des dritten Destillationsstoffgemisches in die erste Destillationskolonne (11) eingeleitet wird.

6. Verfahren nach einem der vorhergehenden Ansprüche, **dadurch gekennzeichnet, dass**
- das Reaktionsgemisch entspannt wird, wodurch ein erstes Entspannungsstoffgemisch entsteht, welches die durch die Entspannung in die Gasphase übergehende Fraktion des Reaktionsgemisches ist, und ein zweites Entspannungsstoffgemisch, welches die in flüssiger Phase verbleibende Fraktion des Reaktionsgemisches ist,
- erstes und zweites Entspannungsstoffgemisch in einem zur Methacroleinaufarbeitungsanlage (110, 210, 310, 410, 510) gehörenden Entspannungsbehälter (5) getrennt werden,
- das erste Entspannungsstoffgemisch in den ersten Kondensator (9) eingeleitet wird und
- das zweite Entspannungsstoffgemisch in die erste Destillationskolonne (11) eingeleitet wird.

7. Verfahren nach Anspruch 6, **dadurch gekennzeichnet, dass** in Schritt S6 wenigstens ein Teil des dritten Destillationsstoffgemisches in den Entspannungsbehälter (5) eingeleitet wird.

8. Verfahren nach einem der vorhergehenden Ansprüche, **dadurch gekennzeichnet, dass** wenigstens ein Teil des in Schritt S3.iv abgeführten zweiten Destillationsstoffgemisches in eine Membrananlage (28) eingeleitet wird, in der Membrananlage (28) wenigstens ein Teil von in dem eingeleiteten zweiten Destillationsstoffgemisch enthaltenem Katalysator zurückgehalten wird und ein Retentatstoffgemisch, welches den zurückgehaltenen Katalysator enthält, sowie ein Permeatstoffgemisch aus der Membrananlage (28) abgeführt werden.

9. Herstellanlage (100, 200, 300, 400, 500, 600, 700, 800, 900) zum Herstellen von Methacrolein aus den Edukten Formaldehyd und Propionaldehyd in Gegenwart von Wasser und in Gegenwart eines homogenen Katalysators, welcher wenigstens auf einer Säure und einer Base basiert, insbesondere Herstellanlage (100, 200, 300, 400, 500, 600, 700, 800, 900) zum Durchführen des Verfahrens nach einem der Ansprüche 1 bis 9, wobei die Herstellanlage (100, 200, 300, 400, 500, 600, 700, 800, 900) aufweist:
- einen Reaktor (1), in welchem eine Reaktion in der Flüssigphase durchgeführt werden kann, bei welcher aus Formaldehyd und Propionaldehyd in Gegenwart von Wasser und in Gegenwart des homogenen Katalysators Methacrolein gebildet wird,
- eine Methacroleinaufarbeitungsanlage (110, 210, 310, 410, 510, 610, 710, 810, 910) zum Verringern des Anteils, den Begleitkomponenten, beispielsweise Wasser, Katalysator, nicht umgesetztes Formaldehyd und nicht umgesetztes Propionaldehyd, an dem Reaktionsgemisch oder an einer Fraktion des Reaktionsgemisches ausmachen, wobei die Methacroleinaufarbeitungsanlage (110, 210, 310, 410, 510, 610, 710, 810, 910) aufweist:
• eine einen Kopf (12) und einen Sumpf (13) aufweisende ersten Destillationskolonne (11), mit welcher zugeführtes Reaktionsgemisch oder eine zugeführte Fraktion von Reaktionsgemisch wenigstens in ein als Gasphase vorliegendes erstes Destillationsstoffgemisch, welches Methacrolein enthält, und ein als flüssige Phase vorliegendes zweites Destillationsstoffgemisch, welches Wasser und Katalysator enthält, getrennt werden kann,
• einen ersten Kondensator (9), mit welchem zugeführtes erstes Destillationsstoffgemisch kondensiert werden kann und welcher einen Kondensatausgang (16) hat,
• einen ersten Phasentrenner (18), mit welchem ein im vom ersten Kondensator (9) zugeführten Kondensat enthaltenes erstes Trennstoffgemisch organischer Phase von einem im vom ersten Kondensator (9) zugeführtem Kondensat enthaltenen zweiten Trennstoffgemisch wässriger Phase getrennt werden kann, wobei der erste Phasentrenner (18) einen ersten Ausgang (19) zum Abführen von erstem Trennstoffgemisch und einen zweiten Ausgang (20) zum Abführen von zweitem Trennstoffgemisch aufweist,
• eine erste Phasentrennerabführleitungsanordnung (21), welche an den ersten Ausgang (19) des ersten Phasentrenners (18) fluidisch angeschlossen ist und durch welche erstes Trennstoffgemisch aus dem ersten Phasentrenner (18) abgeführt werden kann,
• eine zweite Phasentrennerabführleitungsanordnung (22), welche an den zweiten Ausgang (20) des ersten Phasentrenners (18) fluidisch angeschlossen ist und durch welche zweites Trennstoffgemisch aus dem ersten Phasentrenner (18) abgeführt werden kann,
• eine erste Destillationskolonnenabführleitungsanordnung (14), welche den Kopf (12) der ersten Destillationskolonne (11) und den ersten Kondensator (9) fluidisch verbindet und durch welche erstes Destillationsstoffgemisch von der ersten Destillationskolonne (11) in den ersten Kondensator (9) geleitet werden kann,
• eine zweite Destillationskolonnenabführleitungsanordnung (15), welche den Sumpf (13) der ersten Destillationskolonne (11) und den Reaktor (1) fluidisch verbindet und durch welche zweites Destillationsstoffgemisch aus der ersten Destillationskolonne (11) in den Reaktor (1) geleitet werden kann, und
• eine erste Kondensatorabführleitungsanordnung (17), welche den Kondensatausgang (16) des ersten Kondensators (9) und den ersten Phasentrenner (18) fluidisch verbindet und durch welche Kondensat des ersten Kondensators (9) von dem ersten Kondensator (9) in den ersten Phasentrenner (18) geleitet werden kann,
- eine einen Kopf (24) und einen Sumpf (25) aufweisende zweite Destillationskolonne (23), mit welcher zweites Trennstoffgemisch wenigstens in ein als Gasphase vorliegendes und Methacrolein enthaltendes drittes Destillationsstoffgemisch und ein als flüssige Phase vorliegendes und Wasser enthaltendes viertes Destillationsstoffgemisch getrennt werden kann, wobei die zweite Phasentrennerabführleitungsanordnung (22) den zweiten Ausgang (20) des ersten Phasentrenners (18) mit der zweiten Destillationskolonne (23) mit fluidisch verbindet und zweites Trennstoffgemisch durch die zweite Phasentrennerabführleitungsanordnung (22) vom zweiten Ausgang (20) des ersten Phasentrenners (18) in die zweite Destillationskolonne (23) geleitet werden kann,
- eine dritte Destillationskolonnenabführleitungsanordnung (26, 50,70, 80), welche an den Kopf(24) der zweiten Destillationskolonne (23) fluidisch angeschlossen ist und durch welche der Kopf (24) der zweiten Destillationskolonne (23) mit der Methacroleinaufarbeitungsanlage (110, 210, 310, 410, 510, 610, 710, 810, 910) fluidisch verbunden ist, wobei drittes Destillationsstoffgemisch durch die dritte Destillationskolonnenabführleitungsanordnung (26, 50, 70, 80) vom Kopf (24) der zweiten Destillationskolonne (23) in die Methacroleinaufarbeitungsanlage (110, 210, 310, 410, 510, 610, 710, 810, 910) geleitet werden kann, und
- eine vierte Destillationskolonnenabführleitungsanordnung (27), welche an den Sumpf (25) der zweiten Destillationskolonne (23) fluidisch angeschlossen ist und durch welche viertes Destillationsstoffgemisch aus der zweiten Destillationskolonne (23) abgeführt werden kann.

10. Herstellanlage (100, 600) nach Anspruch 9, **dadurch gekennzeichnet, dass** die dritte Destillationskolonnenabführleitungsanordnung (26) den Kopf (24) der zweiten Destillationskolonne (23) mit dem ersten Kondensator (9) fluidisch verbindet und drittes Destillationsstoffgemisch durch die dritte Destillationskolonnenabführleitungsanordnung (26) vom Kopf (24) der zweiten Destillationskolonne (23) in den ersten Kondensator (9) geleitet werden kann.

11. Herstellanlage (200, 700) nach Anspruch 9 oder 10, **dadurch gekennzeichnet,**
- **dass** die Herstellanlage (200, 700) einen zur Methacroleinaufarbeitungsanlage (210, 710) gehörenden zweiten Kondensator (51) aufweist,
- **dass** die dritte Destillationskolonnenabführleitungsanordnung (50) den Kopf (24) der zweiten Destillationskolonne (23) mit dem zweiten Kondensator (51) fluidisch verbindet, wobei drittes Destillationsstoffgemisch durch die dritte Destillationskolonnenabführleitungsanordnung (50) vom Kopf (24) der zweiten Destillationskolonne (23) in den zweiten Kondensator (51) geleitet und in dem zweiten Kondensator (51) kondensiert werden kann und
- **dass** der zweite Kondensator (51) fluidisch mit dem ersten Phasentrenner (18) verbunden ist, wodurch Kondensat des zweiten Kondensators (51) vom zweiten Kondensator (51) weiter in den ersten Phasentrenner (18) geleitet werden kann.

12. Herstellanlage (300, 800) nach einem der Ansprüche 9 bis 11, **dadurch gekennzeichnet,**
- **dass** die Herstellanlage (300, 800) einen zur Methacroleinaufarbeitungsanlage (310, 810) gehörenden zweiten Kondensator (51) aufweist,
- **dass** die dritte Destillationskolonnenabführleitungsanordnung (50) den Kopf (24) der zweiten Destillationskolonne (23) mit dem zweiten Kondensator (51) fluidisch verbindet, wobei drittes Destillationsstoffgemisch durch die dritte Destillationskolonnenabführleitungsanordnung (50) vom Kopf (24) der zweiten Destillationskolonne (23) in den zweiten Kondensator (51) geleitet und in dem zweiten Kondensator (51) kondensiert werden kann, und
- **dass** der zweite Kondensator (51) fluidisch mit einem zur Methacroleinaufarbeitungsanlage (310, 810) gehörenden zweiten Phasentrenner (61) verbunden ist, wodurch Kondensat des zweiten Kondensators (51) vom zweiten Kondensator (51) weiter in den zweiten Phasentrenner (61) geleitet werden kann, mit welchem ein im vom zweiten Kondensator (51) zugeführten Kondensat enthaltenes drittes Trennstoffgemisch organischer Phase von einem im vom zweiten Kondensator (51) zugeführtem Kondensat enthaltenen vierten Trennstoffgemisch wässriger Phase getrennt werden kann, wobei der zweite Phasentrenner (61) einen ersten Ausgang (62) zum Abführen von drittem Trennstoffgemisch und einen zweiten Ausgang (63) zum Abführen von viertem Trennstoffgemisch aufweist.

13. Herstellanlage (400, 900) nach einem der Ansprüche 9 bis 12, **dadurch gekennzeichnet, dass** die dritte Destillationskolonnenabführleitungsanordnung (70) den Kopf (24) der zweiten Destillationskolonne (23) mit der ersten Destillationskolonne (11) fluidisch verbindet und drittes Destillationsstoffgemisch durch die dritte Destillationskolonnenabführleitungsanordnung (70) vom Kopf (24) der zweiten Destillationskolonne (23) in die erste Destillationskolonne (11) geleitet werden kann.

14. Herstellanlage (100, 200, 300, 400, 500) nach einem der Ansprüche 9 bis 13, **dadurch gekennzeichnet, dass** die Methacroleinaufarbeitungsanlage (110, 210, 310, 410, 510) einen Entspannungsbehälter (5) aufweist, mit welchem ein erstes Entspannungsstoffgemisch, welches die durch eine Entspannung des Reaktionsgemisches in die Gasphase übergehende Fraktion des Reaktionsgemisches ist, und ein zweites Entspannungsstoffgemisch, welches die nach der Entspannung des Reaktionsgemisches in flüssiger Phase verbleibende Fraktion des Reaktionsgemisches ist, voneinander getrennt werden können, wobei der Entspannungsbehälter (5) einen ersten Ausgang (6) zum Abführen von erstem Entspannungsstoffgemisch hat, welcher mit dem ersten Kondensator (9) fluidisch verbunden ist, wodurch erstes Entspannungsstoffgemisch in den ersten Kondensator (9) geleitet werden kann, und einen zweiten Ausgang (7) zum Abführen von zweitem Entspannungsstoffgemisch, welcher mit der ersten Destillationskolonne (11) fluidisch verbunden ist, wodurch zweites Entspannungsstoffgemisch in die erste Destillationskolonne (11) geleitet werden kann.

15. Herstellanlage (500) nach Anspruch 14, **dadurch gekennzeichnet, dass** die dritte Destillationskolonnenabführleitungsanordnung (80) den Kopf (24) der zweiten Destillationskolonne (23) mit dem Entspannungsbehälter (5) fluidisch verbindet und drittes Destillationsstoffgemisch durch die dritte Destillationskolonnenabführleitungsanordnung (80) vom Kopf (24) der zweiten Destillationskolonne (23) in den Entspannungsbehälter (5) geleitet werden kann.

16. Herstellanlage (100, 200, 300, 400, 500, 600, 700, 800, 900) nach einem der Ansprüche 9 bis 15, **dadurch gekennzeichnet, dass** der Sumpf (13) der ersten Destillationskolonne (11) mit dem Reaktor (1) fluidisch verbunden ist, wodurch zweites Destillationsstoffgemisch in den Reaktor (1) geleitet werden kann.

17. Herstellanlage (100, 200, 300, 400, 500, 600, 700, 800, 900) nach einem der Ansprüche 9 bis 16, **dadurch gekennzeichnet, dass** die Herstellanlage (100, 200, 300, 400, 500, 600, 700, 800, 900) eine mit dem Sumpf (13) der ersten Destillationskolonne (11) fluidisch verbundene Membrananlage (28) aufweist, mit welcher wenigstens ein Teil von in der Membrananlage zugeleitetem zweiten Destillationsstoffgemisch enthaltenem Katalysator zurückgehalten werden kann, und die Membrananlage (28) einen ersten Ausgang zum Abführen von den zurückgehaltenen Katalysator enthaltendem Retentatstoffgemisch (33) aufweist sowie einen zweiten Ausgang (34) zum Abführen von Permeatstoffgemisch.

18. Verwendung der Herstellanlage (100, 200, 300, 400, 500, 600, 700, 800, 900) nach einem der Ansprüche 9 bis 17 zum Herstellen von Methacrolein, insbesondere zum Herstellen von Methacrolein aus Formaldehyd und Propionaldehyd in Gegenwart von Wasser und in Gegenwart eines homogenen Katalysators, welcher wenigstens auf einer Säure und einer Base basiert.
